(19) Europäisches Patentamt
European Patent Office
Office européen des brevets

(11) EP 4 682 143 A1

(12) EUROPEAN PATENT APPLICATION
published in accordance with Art. 153(4) EPC

(43) Date of publication:
**21.01.2026 Bulletin 2026/04**

(21) Application number: **23926680.2**

(22) Date of filing: **10.03.2023**

(51) International Patent Classification (IPC):
***C07D 401/14*** (2006.01) ***C07D 401/12*** (2006.01)
***A61K 9/16*** (2006.01) ***A61K 31/4709*** (2006.01)
***A61K 31/343*** (2006.01) ***A61K 31/5377*** (2006.01)
***A61P 35/00*** (2006.01)

(52) Cooperative Patent Classification (CPC):
**A61K 9/16; A61K 31/343; A61K 31/4709; A61K 31/5377; A61P 35/00; C07D 401/12; C07D 401/14**

(86) International application number:
**PCT/CN2023/080921**

(87) International publication number:
**WO 2024/187321 (19.09.2024 Gazette 2024/38)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
- **Convalife Pharmaceuticals Co., Ltd.
Shanghai 201318 (CN)**
- **Zhejiang Convalife Pharmaceutical Co., Ltd.
Shaoxing, Zhejiang 312300 (CN)**

(72) Inventors:
- **SHEN, Xiaokun
Shanghai 201318 (CN)**
- **HUANG, Jinwen
Shanghai 201318 (CN)**
- **MING, Congmei
Shanghai 201318 (CN)**
- **SONG, Zeng
Shanghai 201318 (CN)**
- **LI, Zeng
Shanghai 201318 (CN)**

(74) Representative: **Potter Clarkson
Chapel Quarter
Mount Street
Nottingham NG1 6HQ (GB)**

# (54) PHARMACEUTICAL COMPOSITION CONTAINING EGFR INHIBITOR, AND PREPARATION METHOD THEREFOR AND USE THEREOF

(57) Disclosed in the present invention are a pharmaceutical composition containing an EGFR inhibitor, and a preparation method therefor and the use thereof. The pharmaceutical composition contains particles, wherein the particles contain the following components in mass fraction: 30%-45% of anhydrous neratinib maleate, 7%-28% of copovidone, 25%-48% of a filler, 1.0%-8.0% of a disintegrant, 0.5%-4.0% of a glidant and 0.5%-4.0% of a lubricant. The pharmaceutical composition of the present invention has good stability and in-vivo biological activity equivalent to that of a reference preparation, and has a good application prospect.

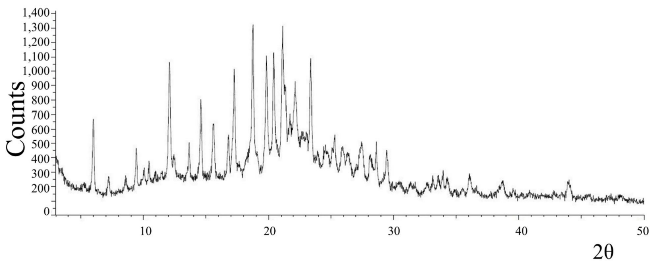


Fig. 1



Processed by Luminess, 75001 PARIS (FR)

## Description

## Technical Field

The present invention relates to a pharmaceutical composition comprising an EGFR inhibitor, as well as a method for its preparation and its use.

## Background Art

(E)-N-(4-(3-chloro-4-(pyridin-2-ylmethoxy)anilino)-3-cyano-7-ethoxyquinolin-6-yl)-4-(dimethylamino)but-2-enamide maleate, also known as neratinib maleate, having the specific structure as shown below, is a specific protein kinase inhibitor.

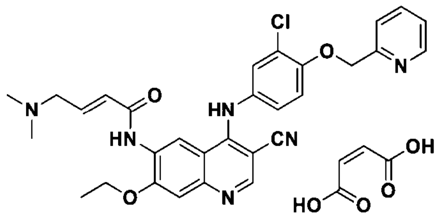

Studies have shown that high expression or abnormal expression of EGFR (epidermal growth factor receptor) exists in many solid tumors. EGFR is associated with tumor cell proliferation, angiogenesis, tumor invasion, metastasis, and inhibition of apoptosis. The overexpression of EGFR plays an important role in the progression of malignant tumors, and such overexpression has been found in gliomas, renal carcinoma, lung cancer, prostate cancer, pancreatic cancer, breast cancer, and other tissues. Elevated levels of EGFR and HER2 have been detected in more than 30% of breast cancer patients, among which HER2 is one of the most important genes in the EGFR family.

The active ingredient of neratinib maleate is neratinib, an irreversible HER2/EGFR inhibitor, which can effectively inhibit the tyrosine kinase activities of HER1 and HER2, thereby suppressing the growth of tumor cells. Neratinib maleate was originally developed by Wyeth, later acquired by Pfizer, and subsequently licensed to Puma Biotechnology, Inc. for further development, commercialization, manufacturing, and sales. On July 17, 2017, neratinib maleate was approved for marketing by the United States Food and Drug Administration (FDA) under the trade name Nerlynx®. Neratinib maleate tablets are prepared from anhydrous crystalline neratinib maleate as the raw material, which is mixed with excipients and processed by a fluidized-bed granulation, tableting, and coating process. During this process, the crystalline form of the active ingredient neratinib maleate is transformed into a hydrated crystalline form.

Although neratinib exhibits potent inhibitory activity against tumor cell growth, it is prone to degradation and suffers from poor stability. Particularly, once formulated into pharmaceutical compositions, the presence of various excipients and water gives rise to the following issues:

1. Neratinib maleate is prone to degradation, and the specific structural formula of its degradation impurity is shown below. This degradation impurity increases rapidly under conditions of high temperature and high humidity, which requires avoiding exposure to heat and water as much as possible during the preparation of the formulation. In clinical application, since degradation impurities of neratinib cause significant side effects, generally, the content of degradation impurities in neratinib formulations needs to be controlled below 1.5%. Therefore, providing a stable neratinib formulation is a challenge for those skilled in the art.

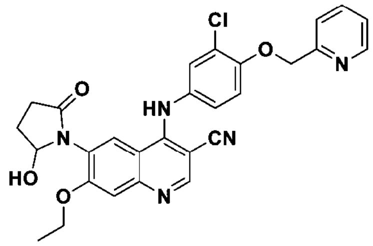

2. Neratinib maleate is easily transformed from the anhydrous crystalline form into the monohydrate crystalline form upon exposure to water. The transformation of the crystalline form affects the control of drug quality, as well as the dissolution and release of the drug, thereby influencing therapeutic efficacy and side effects. Therefore, it is required to

avoid the use of water as much as possible during the preparation of the formulation.

Based on the above two issues, providing a stable neratinib formulation is a significant challenge in the art.

Patent CN102724970 discloses a preparation method of neratinib maleate tablets, which is aimed at improving the binding properties of neratinib during granulation. The disclosed method employs a fluidized-bed wet granulation process, which involves both heat and moisture, without addressing the intrinsic instability of neratinib, which is prone to polymorphic transformation into the hydrated crystalline form and chemical degradation. As such, the final formulation cannot ensure satisfactory stability or dissolution properties. In addition, neratinib maleate undergoes a crystalline transformation from the anhydrous form to the hydrated form during granulation, and the degree of transformation cannot be fully controlled, posing challenges to quality control.

Patent CN106913529 discloses another preparation method of neratinib maleate tablets, in which a fluidized-bed granulation process is adopted, with the water content of the granules being controlled below 10% during the process, and subsequently reduced to less than 2% by drying at high temperature (70-90 °C). Following coating, an additional drying step using a rotary vacuum dryer is required to further reduce the moisture content below 2%. However, such a process is generally not suitable for large-scale industrial production. Moreover, polymorphic transformation of neratinib maleate is still unavoidable, resulting in inconsistent degrees of crystallinity across batches. Additionally, the dissolution rate of the neratinib capsules disclosed therein is too fast (more than 80% within 15 minutes), which is significantly faster than the marketed reference product (original Nerlynx®). Compared with the reference, the disclosed formulation poses a high risk of non-bioequivalence in vivo.

In summary, for the clinical application of neratinib, there is a need to develop a tablet formulation process that avoids the use of water and high temperatures, thereby providing a stable formulation with low impurity levels.

**Summary of the Invention**

The technical problem to be solved by the present invention is to overcome the limitations of the limited variety of pharmaceutical compositions containing EGFR inhibitors in the prior art. To this end, the present invention provides a pharmaceutical composition comprising an EGFR inhibitor, as well as its preparation method and application. The pharmaceutical composition comprising an EGFR inhibitor according to the present invention has good stability and a dissolution profile consistent with that of a reference product, and exhibits in vivo bioactivity equivalent to that of the reference product.

The present invention solves the above technical problem by the following technical solution. The present invention provides a compound represented by the following formula I:

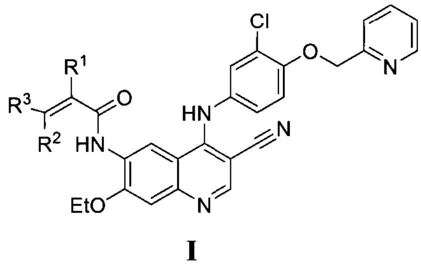

I

,

Wherein $R^1$, $R^2$, and $R^3$ are each independently H or a $C_{1-6}$ alkyl substituted with one or more $R^{1-1}$; each $R^{1-1}$ is independently a 4-6 membered heterocycloalkyl in which the heteroatoms are selected from one, two, or three of N, O, and S, with one, two, or three heteroatoms, a 4-6 membered heterocycloalkyl substituted with one or more $R^{1-a}$ in which the heteroatoms are selected from one, two, or three of N, O, and S, with one, two, or three heteroatoms, or $-SO_2-C_{1-6}$ alkyl; each $R^{1-a}$ is independently halogen or $-N(C_{1-6}$ alkyl$)_2$.

In some embodiments, $R^1$ and $R^2$ are H, and $R^3$ is a $C_{1-6}$ alkyl substituted with one or more $R^{1-1}$. In some embodiments, $R^2$ and $R^3$ are H, and $R^1$ is a $C_{1-6}$ alkyl substituted with one or more $R^{1-1}$. In some embodiments, each $R^{1-1}$ is independently a 4-6 membered heterocycloalkyl substituted with one or more $R^{1-a}$ in which the heteroatoms are selected from one, two, or three of N, O, and S, with one, two, or three heteroatoms, or $-SO_2-C_{1-6}$ alkyl.

In some embodiments, in $R^1$, $R^2$, $R^3$, $R^{1-1}$, and $R^{1-a}$, the $C_{1-6}$ alkyl in "$C_{1-6}$ alkyl substituted with one or more $R^{1-1}$," "$-SO_2-C_{1-6}$ alkyl," and "$-N(C_{1-6}$ alkyl$)_2$" are each independently methyl, ethyl, n-propyl, isopropyl, n-butyl, isobutyl, or tert-butyl, preferably methyl.

In some embodiments, the "4-6 membered heterocycloalkyl in which the heteroatoms are selected from one, two, or three of N, O, and S, with one, two, or three heteroatoms" of the 4-6 membered heterocycloalkyl substituted with one or more $R^{1-a}$ is independently a

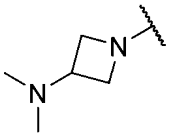,

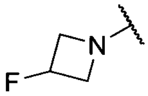, or 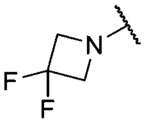.

In some embodiments, in $R^{1-1}$, the "4-6 membered heterocycloalkyl in which the heteroatoms are selected from one, two, or three of N, O, and S, with one, two, or three heteroatoms" in the "4-6 membered heterocycloalkyl in which the heteroatoms are selected from one, two, or three of N, O, and S, with one, two, or three heteroatoms" and in the "4-6 membered heterocycloalkyl substituted with one or more $R^{1-a}$ in which the heteroatoms are selected from one, two, or three of N, O, and S, with one, two, or three heteroatoms" are each independently a 4-6 membered heterocycloalkyl containing one N heteroatom, preferably

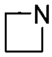 or 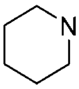.

In some embodiments, in $R^{1-a}$, the halogen is fluorine, chlorine, bromine, or iodine, preferably fluorine.

In some embodiments, $R^1$ and $R^2$ are H, and $R^3$ is

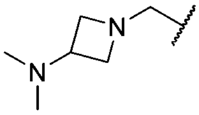, 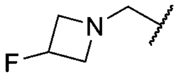,

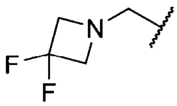 or 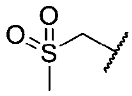.

In some embodiments, $R^2$ and $R^3$ are H, and $R^1$ is

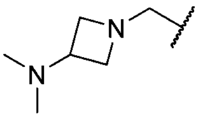 or 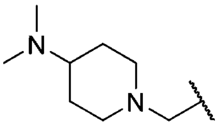.

In some embodiments, the compound of formula I is any one of the following compounds:

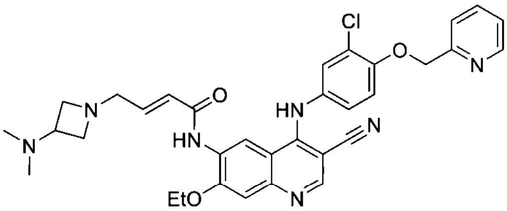 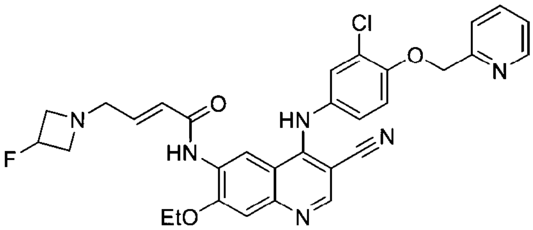

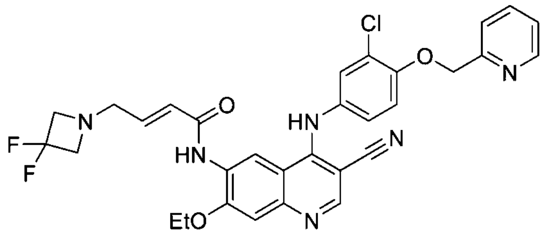

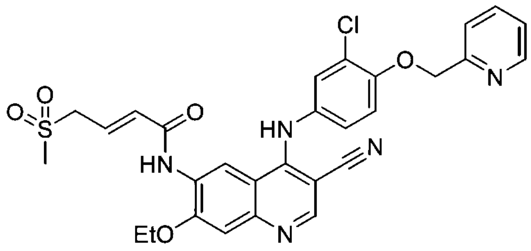

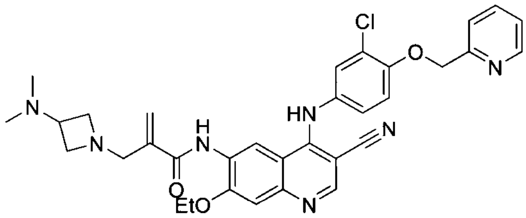

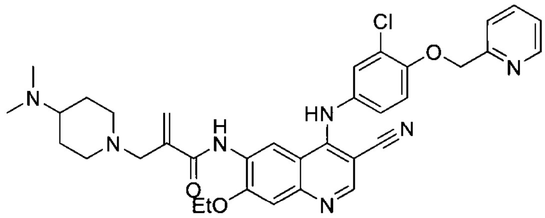

.

The present invention further provides a pharmaceutical composition 1, comprising a compound of formula I or a pharmaceutically acceptable salt thereof, and a pharmaceutically acceptable excipient.

The present invention further provides the use of a compound of formula I or a pharmaceutically acceptable salt thereof in the preparation of a medicament for preventing or treating an EGFR-related disease.

The EGFR-related disease is preferably breast cancer, ovarian cancer, epithelial tumor, colon cancer, prostate cancer, kidney cancer, bladder cancer, laryngeal cancer, esophageal cancer, gastric cancer, or lung cancer.

The present invention further provides a pharmaceutical composition 2, comprising particles, wherein the particles comprise the following components in the following mass fractions: 30-45% of a compound of formula II or a pharmaceutically acceptable salt thereof, 7-28% of copovidone, 25-48% of a filler, 1.0-8.0% of a disintegrant, 0.5-4.0% of a glidant, and 0.5-4.0% of a lubricant; the sum of the mass fractions of all components in the particles is 100%;

the mass fraction refers to the percentage by mass of each component in the particles relative to the total mass of all components;

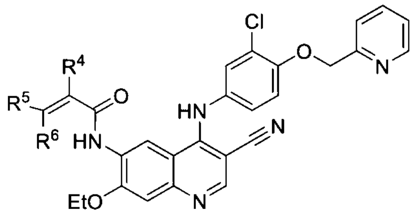

**II**

;

$R^4$, $R^5$, and $R^6$ are each independently H or a $C_{1-6}$ alkyl substituted with one or more $R^{4-1}$;
each $R^{4-1}$ is independently -N($C_{1-6}$ alkyl)$_2$, a 4-6 membered heterocycloalkyl in which the heteroatoms are selected from one, two, or three of N, O, and S, with one, two, or three heteroatoms, a 4-6 membered heterocycloalkyl substituted with one or more $R^{4-a}$ in which the heteroatoms are selected from one, two, or three of N, O, and S, with one, two, or three heteroatoms, or -$SO_2$-$C_{1-6}$ alkyl;
each $R^{4-a}$ is independently halogen or -N($C_{1-6}$ alkyl)$_2$.

In some embodiments, $R^4$ and $R^6$ are H, and $R^5$ is a $C_{1-6}$ alkyl substituted with one or more $R^{4-1}$.

In some embodiments, $R^5$ and $R^6$ are H, and $R^4$ is a $C_{1-6}$ alkyl substituted with one or more $R^{4-1}$.

In some embodiments, each $R^{4-1}$ is independently -N($C_{1-6}$ alkyl)$_2$, a 4-6 membered heterocycloalkyl substituted with one or more $R^{4-a}$ in which the heteroatoms are selected from one, two, or three of N, O, and S, with one, two, or three heteroatoms, or -$SO_2$-$C_{1-6}$ alkyl.

In some embodiments, in $R^4$, $R^5$, $R^6$, $R^{4-1}$, and $R^{4-a}$, the $C_{1-6}$ alkyl in "$C_{1-6}$ alkyl substituted with one or more $R^{4-1}$," "-$SO_2$-$C_{1-6}$ alkyl," and "-N($C_{1-6}$ alkyl)$_2$" are each independently methyl, ethyl, n-propyl, isopropyl, n-butyl, isobutyl, or tert-butyl, preferably methyl.

In some embodiments, in $R^{4-1}$, the 4-6 membered heterocycloalkyl in which the heteroatoms are selected from one, two, or three of N, O, and S, with one, two, or three heteroatoms, and in the 4-6 membered heterocycloalkyl substituted

with one or more in which the heteroatoms are selected from one, two, or three of N, O, and S, with one, two, or three heteroatoms, are each independently a 4-6 membered heterocycloalkyl containing one N heteroatom, preferably

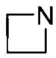 or 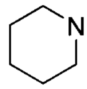 .

In some embodiments, in $R^{4-a}$, the halogen is fluorine, chlorine, bromine, or iodine, preferably fluorine.

In some embodiments, $R^4$ and $R^6$ are H, and $R^5$ is

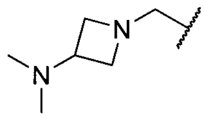 , 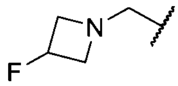 ,

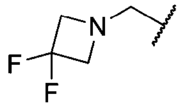 , 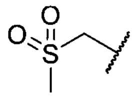 or 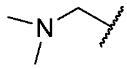 .

In some embodiments, $R^5$ and $R^6$ are H, and $R^4$ is

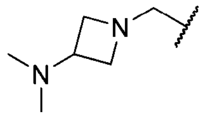 or 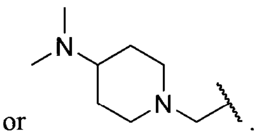 .

In some embodiments, the compound of formula II is any one of the following compounds:

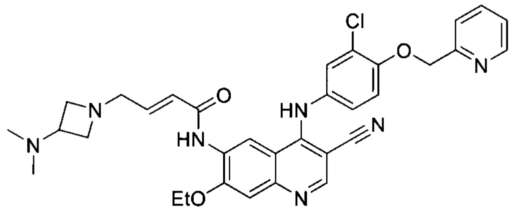 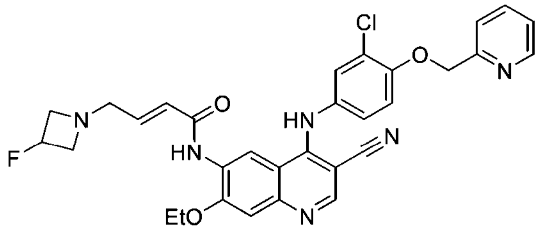

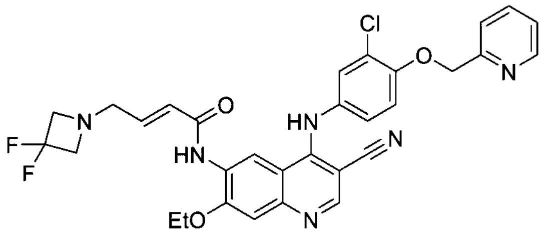 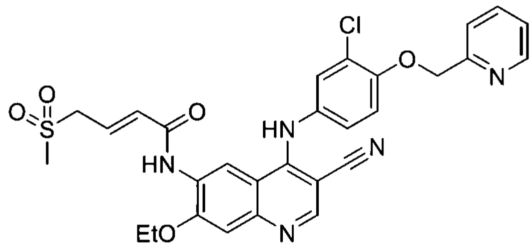

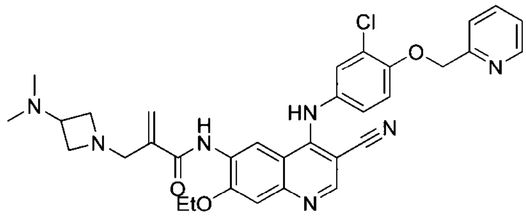 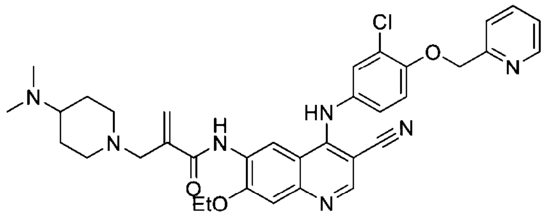

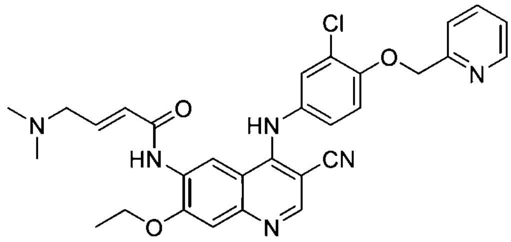
.

In some embodiments, the pharmaceutically acceptable salt is the maleate salt of the compound of formula II.
In some embodiments, the pharmaceutically acceptable salt exists in an anhydrous crystalline or amorphous form, preferably in an anhydrous crystalline form.
In some embodiments, the mass fraction of the compound of formula II or its pharmaceutically acceptable salt can be 35-42%, for example 36%.
In some embodiments, the mass fraction of copovidone can be 8-22%, for example 21%.
In some embodiments, the filler can be a conventional filler in the pharmaceutical field, preferably a sugar alcohol and/or a water-swellable additive, more preferably mannitol and microcrystalline cellulose.
The sugar alcohol is preferably one or more of mannitol, crythritol, and xylitol, for example mannitol.
The water-swellable filler is preferably one or more of pregelatinized starch, gelatinized starch, microcrystalline cellulose (crystalline cellulose), corn starch, hydroxypropyl methylcellulose (HPMC-K100LV), calcium sulfate, sodium carboxymethyl starch, carboxymethyl cellulose, calcium carboxymethyl cellulose, crosslinked sodium carboxymethyl cellulose, soybean lecithin, low-substituted hydroxypropyl cellulose, tragacanth gum powder, and bentonite, for example microcrystalline cellulose.
In some embodiments, the mass fraction of the filler can be 34-45%, for example 37%.
In some embodiments, the disintegrant can be a conventional disintegrant in the pharmaceutical field, preferably one or more of adipic acid, alginic acid, gelatinized starch, sodium carboxymethyl starch, carboxymethyl cellulose, calcium carboxymethyl cellulose, sodium carboxymethyl cellulose, hydrated silicon dioxide, calcium citrate, crosslinked sodium carboxymethyl cellulose, crospovidone, light anhydrous silica, crystalline cellulose (microcrystalline cellulose), synthetic aluminum silicate, wheat starch, rice starch, cellulose acetate phthalate, calcium stearate, low-substituted hydroxypropyl cellulose, corn starch, tragacanth gum powder, potato starch, hydroxyethyl methylcellulose, hydroxypropyl starch, pregelatinized starch, monosodium fumarate, povidone, anhydrous citric acid, methylcellulose, and dicalcium phosphate, more preferably crospovidone, for example crospovidone XL-10.
In some embodiments, the mass fraction of the disintegrant can be 1.7-4.9%, for example 2.0%.
In some embodiments, the glidant can be a conventional glidant in the pharmaceutical field, preferably one or more of hydrated silicon dioxide (colloidal silicon dioxide), gel silicon dioxide, light anhydrous silica, crystalline cellulose, synthetic aluminum silicate, titanium dioxide, stearic acid, calcium stearate, magnesium stearate, tricalcium phosphate, talc, corn starch, and magnesium aluminosilicate, preferably colloidal silicon dioxide or gel silicon dioxide.
In some embodiments, the amount of the glidant can be a conventional amount in the pharmaceutical field. The mass fraction of the glidant can be 1.0-4.0%, for example 2.0%.
In some embodiments, the lubricant can be a conventional lubricant in the pharmaceutical field, preferably one or more of cocoa butter, Brazilian palm wax, hydrated silicon dioxide (colloidal silicon dioxide), aluminum hydroxide dry gel, glycerol esters of fatty acids, magnesium silicate, light anhydrous silica, crystalline cellulose, hydrogenated oil, synthetic aluminum silicate, white beeswax, magnesium oxide, sodium potassium tartrate, sucrose esters of fatty acids, stearic acid, calcium stearate, magnesium stearate, sodium stearyl fumarate, stearyl alcohol, and polyethylene glycol 40 stearate, preferably sodium stearyl fumarate or magnesium stearate.
In some embodiments, the mass fraction of the lubricant can be 1.0-4.0%, for example 2%.
In some embodiments, the pharmaceutical composition 2 further comprises a coating agent.
In some embodiments, the coating agent can be a conventional coating agent used in such pharmaceutical compositions, preferably one or more of hydroxypropyl methylcellulose, methylcellulose, ethylcellulose, methylcellulose or hydroxypropyl cellulose, polyvinyl alcohol, povidone, polyvinyl acetate resin, polyvinyl alcohol phthalate diethylaminoacetate, methacrylic acid aminoalkyl ester copolymer RS, ethyl acrylate-methyl methacrylate copolymer dispersion, sucrose, mannitol, and Opadry® (commercial name), preferably Opadry®.
In some embodiments, the pharmaceutical composition 2 comprises particles, wherein the particles comprise the following components in the following mass fractions: 30-45% anhydrous neratinib maleate, 7-28% copovidone, 25-48% filler, 1.0-8.0% disintegrant, 0.5-4.0% glidant, and 0.5-4.0% lubricant.
The sum of the mass fractions of all components in the particles is 100%; the mass fraction refers to the percentage by mass of each component in the particles relative to the total mass of all components.
In the pharmaceutical composition 2, the anhydrous neratinib maleate can be in a crystalline form of anhydrous

neratinib maleate, wherein the X-ray powder diffraction (XRPD) pattern, represented by diffraction angles 2θ, shows characteristic peaks at 6.0±0.2°, 7.3+0.2°, 10.1±0.2°, 12.1±0.2°, 15.6±0.2°, 17.3±0.2°, and 19.9±0.2°. The XRPD pattern of the crystalline form of neratinib maleate can be substantially as shown in Figures 1, 2, or 3.

In the pharmaceutical composition 2, the mass fraction of anhydrous neratinib maleate can be 35-42%, for example 36%.

In the pharmaceutical composition 2, the mass fraction of copovidone can be 8-22%, for example 21%.

In the pharmaceutical composition 2, the filler can be a conventional filler in the pharmaceutical field, preferably a sugar alcohol and/or a water-swellable additive, more preferably mannitol and microcrystalline cellulose.

The sugar alcohol is preferably one or more of mannitol, erythritol, and xylitol, for example mannitol.

The water-swellable filler is preferably one or more of pregelatinized starch, gelatinized starch, microcrystalline cellulose (crystalline cellulose), corn starch, hydroxypropyl methylcellulose (HPMC-K100LV), calcium sulfate, sodium carboxymethyl starch, carboxymethyl cellulose, calcium carboxymethyl cellulose, crosslinked sodium carboxymethyl cellulose, soybean lecithin, low-substituted hydroxypropyl cellulose, tragacanth gum powder, and bentonite, for example microcrystalline cellulose.

In the pharmaceutical composition 2, the mass fraction of the filler can be 34-45%, for example 37%.

In the pharmaceutical composition 2, the disintegrant can be a conventional disintegrant in the pharmaceutical field, preferably one or more of adipic acid, alginate, gelatinized starch, sodium carboxymethyl starch, carboxymethyl cellulose, calcium carboxymethyl cellulose, sodium carboxymethyl cellulose, hydrated silicon dioxide, calcium citrate, crosslinked sodium carboxymethyl cellulose, crospovidone, light anhydrous silica, crystalline cellulose (microcrystalline cellulose), synthetic aluminum silicate, wheat starch, rice starch, cellulose acetate phthalate, calcium stearate, low-substituted hydroxypropyl cellulose, corn starch, tragacanth gum powder, potato starch, hydroxyethyl methylcellulose, hydroxypropyl starch, pregelatinized starch, monosodium fumarate, povidone, anhydrous citric acid, methylcellulose, and dicalcium phosphate, more preferably crospovidone, for example crospovidone XL-10.

In the pharmaceutical composition 2, the mass fraction of the disintegrant can be 1.7-4.9%, for example 2.0%.

In the pharmaceutical composition 2, the glidant can be a conventional glidant in the pharmaceutical field, preferably one or more of hydrated silicon dioxide (colloidal silicon dioxide), gel silicon dioxide, light anhydrous silica, crystalline cellulose, synthetic aluminum silicate, titanium dioxide, stearic acid, calcium stearate, magnesium stearate, tricalcium phosphate, talc, corn starch, and magnesium aluminosilicate, preferably colloidal silicon dioxide or gel silicon dioxide.

In the pharmaceutical composition 2, the amount of the glidant can be a conventional amount in the pharmaceutical field. The mass fraction of the glidant can be 1.0-4.0%, for example 2.0%.

In the pharmaceutical composition 2, the lubricant can be a conventional lubricant in the pharmaceutical field, preferably one or more of cocoa butter, Brazilian palm wax, hydrated silicon dioxide (colloidal silicon dioxide), aluminum hydroxide dry gel, glycerol esters of fatty acids, magnesium silicate, light anhydrous silica, crystalline cellulose, hydrogenated oil, synthetic aluminum silicate, white beeswax, magnesium oxide, sodium potassium tartrate, sucrose esters of fatty acids, stearic acid, calcium stearate, magnesium stearate, sodium stearyl fumarate, stearyl alcohol, and polyethylene glycol 40 stearate, preferably sodium stearyl fumarate or magnesium stearate.

In the pharmaceutical composition 2, the mass fraction of the lubricant can be 1.0-4.0%, for example 2%.

In the pharmaceutical composition 2, the components of the particles preferably comprise inner components and outer components of the particles;
The inner components of the particles: 30-45% anhydrous neratinib maleate (for example 35%, 36%, or 42%), 7-28% copovidone (for example 8%, 21%, or 22%), 25-48% filler (for example 34%, 37%, or 45%), 1.0-8.0% disintegrant (for example 2.4%), 1.0-4.0% glidant (for example 1%, 2%, or 4%), and 0.5-4.0% lubricant (for example 1%).

The outer components of the particles: 0-2.5% disintegrant and/or 0-1% lubricant, wherein the disintegrant and the lubricant are not simultaneously 0.

In one embodiment, the particles comprise the following components in the following mass fractions: the aforementioned anhydrous neratinib maleate (including the type and mass fraction of neratinib maleate), the aforementioned copovidone, the aforementioned filler (including the type and mass fraction of the filler), the aforementioned disintegrant (including the type and mass fraction of the disintegrant), the aforementioned glidant (including the type and mass fraction of the glidant), the aforementioned lubricant (including the type and mass fraction of the lubricant), and the aforementioned coating agent (including the type and mass fraction of the coating agent).

Preferably, the particles comprise components from group (1), group (2), or group (3):

Group (1):

Inner components of the particles: 34.9% anhydrous neratinib maleate, 26.2% mannitol, 8.3% microcrystalline cellulose, 21.7% copovidone, 2.5% crospovidone XL-10, 2.0% colloidal silicon dioxide, and 1.0% magnesium stearate;

Outer components of the particles: 2.5% crospovidone XL-10 and 1.0% magnesium stearate;

Group (2):

Inner components of the particles: 42.3% anhydrous neratinib maleate, 31.7% mannitol, 13.1% microcrystalline cellulose, 8.0% copovidone, 2.0% crospovidone XL-10, 2.0% colloidal silicon dioxide, and 1.0% magnesium stearate;
Outer components of the particles: 1.0% magnesium stearate;

Group (3):

Inner components of the particles: 36.1% anhydrous neratinib maleate, 27.1% mannitol, 8.6% microcrystalline cellulose, 22.4% copovidone, 1.7% crospovidone XL-10, 2.0% colloidal silicon dioxide, and 1.0% magnesium stearate;
Outer components of the particles: 1.0% magnesium stearate;

The pharmaceutical composition 2 may further comprise a coating agent.
The coating agent can be a conventional coating agent used in such pharmaceuticals in the art, preferably one or more of hydroxypropyl methylcellulose, methylcellulose, ethylcellulose, methylcellulose or hydroxypropyl cellulose, polyvinyl alcohol, povidone, polyvinyl acetate resin, polyvinyl alcohol-acetal diethylaminoacetate, methacrylic acid aminoalkyl ester copolymer RS, ethyl acrylate-methyl methacrylate copolymer dispersion, sucrose, mannitol, and Opadry® (trade name), preferably Opadry®.
The amount of the coating agent can be a conventional amount used in such pharmaceuticals in the art. The mass ratio of the coating agent to the pharmaceutical particles can be 0.01:1-0.05:1, for example 0.03:1, 0.034:1, or 0.036:1.
In the pharmaceutical composition 2, the particle components comprise the following components in the following mass fractions: the aforementioned anhydrous neratinib maleate (including the type and mass fraction of neratinib maleate), the aforementioned copovidone, the aforementioned filler (including the type and mass fraction of the filler), the aforementioned disintegrant (including the type and mass fraction of the disintegrant), the aforementioned glidant (including the type and mass fraction of the glidant), the aforementioned lubricant (including the type and mass fraction of the lubricant), and the aforementioned coating agent (including the type and mass fraction of the coating agent).
The pharmaceutical composition 2 comprises components from group (a), group (b), or group (c):

Group (a): particles and Opadry®, wherein the mass ratio of Opadry® to the particles is 0.03:1; Inner components of the particles: 34.9% anhydrous neratinib maleate, 26.2% mannitol, 8.3% microcrystalline cellulose, 21.7% copovidone, 2.5% crospovidone XL-10, 2.0% colloidal silicon dioxide, and 1.0% magnesium stearate;
Outer components of the particles: 2.5% crospovidone XL-10 and 1.0% magnesium stearate;
Group (b): particles and Opadry®, wherein the mass ratio of Opadry® to the particles is 0.034:1; Inner components of the particles: 42.3% anhydrous neratinib maleate, 31.7% mannitol, 13.1% microcrystalline cellulose, 8.0% copovidone, 2.0% crospovidone XL-10, 2.0% colloidal silicon dioxide, and 1.0% magnesium stearate;
Outer components of the particles: 1.0% magnesium stearate;
Group (c): particles and Opadry®, wherein the mass ratio of Opadry® to the particles is 0.036:1; Inner components of the particles: 36.1% anhydrous neratinib maleate, 27.1% mannitol, 8.6% microcrystalline cellulose, 22.4% copovidone, 1.7% crospovidone XL-10, 2.0% colloidal silicon dioxide, and 1.0% magnesium stearate;
Outer components of the particles: 1.0% magnesium stearate.

The present invention also provides a method for preparing the above-described pharmaceutical composition 2, comprising the following steps: granulating the following raw materials in the indicated mass fractions to obtain particles;
The raw materials comprise 30-45% anhydrous neratinib maleate, 7-28% copovidone, 25-48% filler, 1.0-8.0% disintegrant, 0.5-4.0% glidant, and 0.5-4.0% lubricant; the sum of the mass fractions of all components in the particles is 100%; the mass fraction refers to the mass percentage of each component in the total mass of all components in the particles.
In the present invention, the neratinib maleate is preferably anhydrous neratinib maleate, as previously described (including type and content).
In the present invention, the copovidone, the filler (including type and content), the disintegrant (including type and content), the glidant (including type and content), and the lubricant (including type and content) are as previously described.
In the present invention, the granulation method can be a conventional method in the field, preferably dry granulation. The conditions and operations of the dry granulation are conventional in the field of formulations. The present

invention particularly preferably employs the following conditions and operations:
In the dry granulation, a dry granulator is used, preferably with the following parameters:
The roller pressure of the dry granulator is preferably 65-100 bar, for example, 80 bar.
The feeding speed of the dry granulator is preferably 40-50 rpm.
The roller speed of the dry granulator is preferably 5-15 rpm, for example, 6 rpm.
The compaction speed of the dry granulator is preferably 50-500 rpm, for example, 100 rpm.
The mesh size of the dry granulator is preferably 0.6-1.2 mm, for example, 0.8 mm.
The method for preparing the pharmaceutical composition may further comprise a coating step.
In the coating step, the water content of the pharmaceutical composition is ≤3%. The type and amount of the coating agent are as previously described.
In the coating step, a coating machine is used, preferably with the following parameters:
The inlet air temperature of the coating machine is preferably 55-60°C.
The atomization pressure of the coating machine is preferably 0.10-0.30 MPa.
The peristaltic pump speed of the coating machine is preferably 2.0-12.0 rpm.
The outlet air temperature of the coating machine is preferably 35-42°C.
In one embodiment of the present invention, the method for preparing the pharmaceutical composition 2 preferably comprises the following steps:

Step 1: Granulating the internal raw materials of the granules by dry granulation to obtain granules 1;
Internal raw materials of the granules: 30-45% anhydrous neratinib maleate, 7-28% copovidone, 25-48% filler, 1-8% disintegrant, 1-4% glidant, and 0.5-4% lubricant;
Step 2: Tableting granules 1 with the external raw materials of the granules (for example, mixing granules 1 with the external raw materials of granules 1, and compressing the mixture into tablets using a tablet press (10.5 mm × 5.5 mm shaped punch)) to obtain granules;
External raw materials of the granules: 0-2.5% disintegrant and/or 0-1% lubricant, wherein the disintegrant and the lubricant are not simultaneously 0.

The present invention also provides a method for preparing the pharmaceutical composition 2 described above, thereby obtaining the pharmaceutical composition 2.
The present invention further provides a solid pharmaceutical formulation, which comprises the above pharmaceutical composition 2 and pharmaceutically acceptable excipients.
The solid formulation may be in the form of a tablet, granule, powder (including fine granules), or capsule, for example, a tablet.
When the solid formulation is a tablet, and the pharmaceutical composition 2 of the present invention is in tablet form, the tablet can be obtained by compressing the above-prepared granules. The compression pressure may be 10-20 kN. The shape of the tablet is not particularly limited and may, for example, be lenticular, discoid, round, oval (such as a caplet), teardrop-shaped, or polygonal (such as triangular or rhomboid).
The prepared tablets can be coated by spraying a suspension or solution of a coating agent using a pan coater. After coating, the moisture content of the final tablets can be controlled to ≤3% through a drying process. The drying temperature may be selected from 40-80°C, for example, 40-50°C. During the compression process, the environmental humidity should be controlled to ensure that the moisture content of the final uncoated tablets is less than 3%, and vacuum drying can be applied to the final composition to ensure that its moisture content is less than 3%.
The present invention also provides the use of substance A in the preparation of an EGFR inhibitor, wherein the substance A is the above-mentioned pharmaceutical composition 2 or the above-mentioned solid pharmaceutical formulation.
In some embodiments, the substance A may also be the compound as shown in Formula I or a pharmaceutically acceptable salt thereof.
The present invention further provides the use of substance A in the preparation of a medicament for preventing or treating EGFR-related diseases, wherein the substance A is the above-mentioned pharmaceutical composition 2 or the above-mentioned solid pharmaceutical formulation.
In some embodiments, the substance A may also be the compound as shown in Formula I or a pharmaceutically acceptable salt thereof.
The EGFR-related disease is preferably breast cancer, ovarian cancer, epithelial tumor, colon cancer, prostate cancer, kidney cancer, bladder cancer, laryngeal cancer, esophageal cancer, gastric cancer, or lung cancer.
The present invention also provides a pharmaceutical composition 3, which comprises substance X and substance Y;
wherein substance X is neratinib maleate, and substance Y is compound CVL218 or a pharmaceutically acceptable salt thereof, and the structure of compound CVL218 is as follows:

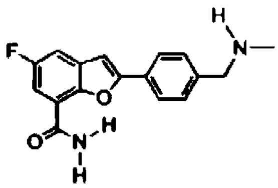

The present invention also provides a pharmaceutical composition 4, which comprises substance M and substance N;
wherein substance M is neratinib maleate, and substance N is compound CVL237 or a pharmaceutically acceptable salt thereof, the structure of compound CVL237 being as follows:

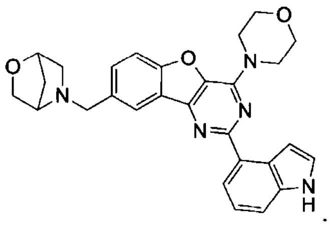

The present invention also provides the use of pharmaceutical composition 3 in the preparation of a medicament for preventing or treating cholangiocarcinoma.
The present invention also provides the use of pharmaceutical composition 4 in the preparation of a medicament for preventing or treating cholangiocarcinoma.
In the present invention, the term "pharmaceutically acceptable salt" refers to a salt of a compound of the invention prepared with a relatively non-toxic, pharmaceutically acceptable acid.
In the present invention, the term "granules" refers to particles having a particle size between 30 mesh and 200 mesh.
In the present invention, the term "pharmaceutically acceptable excipient" refers to those excipients widely used in the field of pharmaceutical manufacturing. Excipients are primarily used to provide a safe, stable, and functional pharmaceutical composition, and may also provide means for the active ingredient to be released at a desired rate after administration to a subject, or to facilitate effective absorption of the active ingredient upon administration. The pharmaceutically acceptable excipients may be inert fillers or may provide certain functions, such as stabilizing the overall pH of the composition or preventing degradation of the active ingredient. The pharmaceutically acceptable excipients may include one or more of the following excipients: binders, suspending agents, emulsifiers, diluents, fillers, granulating agents, adhesives, disintegrants, lubricants, anti-adherents, glidants, wetting agents, gelling agents, absorption retardants, dissolution inhibitors, enhancers, adsorbents, buffers, chelating agents, preservatives, colorants, flavor correctives, and sweeteners.
Without departing from common knowledge in the field, the above-described preferred conditions may be combined in any manner to obtain various preferred embodiments of the present invention.
The reagents and raw materials used in the present invention are all commercially available.
The neratinib or its pharmaceutically acceptable salts or solvates used in the present invention can be obtained by the methods described in US6002008, US6288082, US6297258, US6384051, and US7399865, or purchased through commercial sources.
The neratinib maleate reference product used in the present invention is the original branded neratinib maleate tablet produced by Puma Biotechnology.
The advantageous technical effects of the present invention are that the pharmaceutical compositions of the present invention exhibit good stability and achieve in vitro dissolution profiles comparable to those of the original reference product. Furthermore, the preparation method of the present invention provides products with uniform and stable particle size distribution, minimal tablet weight variation, and easily controllable tablet hardness.

**Brief Description of the Drawings**

Figure 1. XRPD pattern of Example 1 (anhydrous form).
Figure 2. XRPD pattern of Example 2 (anhydrous form).
Figure 3. XRPD pattern of Example 3 (anhydrous form).

Figure 4. XRPD pattern of the reference product (hydrate form).
Figure 5. Dissolution profiles of Example 1, Example 2, and the reference product.
Figure 6. XRPD pattern of Example 4 (anhydrous form).
Figure 7. XRPD pattern of Scale-up Batch 1 in Example 5 (anhydrous form).
Figure 8. XRPD pattern of Scale-up Batch 2 in Example 5 (anhydrous form).
Figure 9. XRPD pattern of Scale-up Batch 3 in Example 5 (anhydrous form).
Figure 10. XRPD pattern of Scale-up Batch 1 in Example 5 after 6 months of accelerated testing (anhydrous form).
Figure 11. XRPD pattern of Scale-up Batch 2 in Example 5 after 6 months of accelerated testing (anhydrous form).
Figure 12. XRPD pattern of Scale-up Batch 3 in Example 5 after 6 months of accelerated testing (anhydrous form).
Figure 13. XRPD pattern of Scale-up Batch 1 in Example 5 after 12 months of long-term testing (anhydrous form).
Figure 14. XRPD pattern of Scale-up Batch 2 in Example 5 after 12 months of long-term testing (anhydrous form).
Figure 15. XRPD pattern of Scale-up Batch 3 in Example 5 after 12 months of long-term testing (anhydrous form).
Figure 16. Dissolution profiles of the Scale-up Batches in Example 5 and the reference product.
Figure 17. Dissolution profiles of the Scale-up Batches in Example 5 after 6 months of accelerated testing and the reference product.
Figure 18. Dissolution profiles of the Scale-up Batches in Example 5 after 12 months of long-term testing.
Figure 19. Body weight change curves of mice in the biological examples.
Figure 20. Tumor volume change curves in the biological examples.

## Detailed Description of the Invention

The present invention is further illustrated by the following examples, which are not intended to limit the scope of the invention to the embodiments described herein. Experimental methods for which specific conditions are not indicated in the following examples are carried out according to conventional methods and conditions, or in accordance with the instructions provided in commercial manuals.

### Preparation Example 1

### Synthesis of (E)-N-(4-((3-chloro-4-(pyridin-2-ylmethoxy)phenyl)amino)-3-cyano-7-ethoxyquinolin-6-yl)-4-(3-(dimethylamino)azetidin-1-yl)but-2-enamide (Compound A0901)

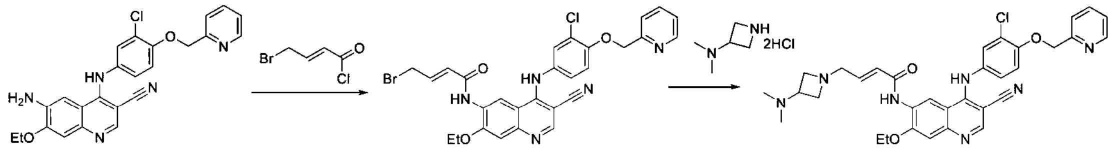

1

2

CVL-A0901

**Step 1:**

**(E)-4-bromo-N-(4-((3-chloro-4-(pyridin-2-ylmethoxy)phenyl)amino)-3-cyano-7-ethoxyquinolin-6-yl)but-2-enamide**

(E)-4-bromobut-2-enoic acid (1.1 g, 6.70 mmol) was dissolved in dichloromethane (10 mL) and cooled in an ice bath. Oxalyl chloride (850 mg, 6.70 mmol) was added, and the reaction mixture was stirred at 10 °C for 4 hours. The resulting solution was then added to a mixture of 6-amino-4-{[3-chloro-4-(pyridin-2-ylmethoxy)phenyl]amino}-7-ethoxyquinoline-3-carbonitrile (2.5 g, 5.60 mmol) and potassium carbonate (2.32 g, 16.8 mmol) in acetonitrile (50 mL). The reaction was stirred at 10 °C for 1 hour. The mixture was poured into water (100 mL) and extracted with ethyl acetate (150 mL × 2). The combined organic layers were washed with brine (100 mL), dried over anhydrous sodium sulfate, filtered, and concentrated to afford the desired intermediate compound 2 (2.30 g) as a yellow solid. LC-MS (ESI, m/z) 592.1 $[M+H]^+$. $^1$HNMR (400 MHz, DMSO-$d_6$) δ 9.94 - 9.70 (m,2H), 9.00 - 8.89 (m, 1H), 8.61 - 8.53 (m, 2H), 7.90 - 7.86 (m, 1H), 7.59 (d, $J$ = 7.6 Hz, 1H), 7.44 - 7.34 (m, 3H), 7.29 - 7.22 (m, 2H), 6.96 - 6.70 (m, 1H), 5.34 (s, 2H), 4.35 - 4.27 (m, 2H), 3.36 (br s, 2H), 1.47 (t, $J$ = 6.8 Hz, 3H).

**Step 2: (E)-N-(4-((3-chloro-4-(pyridin-2-ylmethoxy)phenyl)amino)-3-cyano-7-ethoxyquinolin-6-yl)-4-(3-(dimethylamino)azetidin-1-yl)but-2-enamide**

To a solution of (2E)-4-bromo-N-(4-{[3-chloro-4-(pyridin-2-ylmethoxy)phenyl]amino } -3-cyano-7-ethoxyquinolin-6-yl)but-2-enamide (100 mg, 0.169 mmol) in DMF (3 mL) were added N,N-dimethylazetidin-3-amine hydrochloride (58.4 mg, 0.337 mmol) and ethyldiisopropylamine (65.4 mg, 0.16 mmol). The reaction mixture was poured into water (10 mL) and extracted with ethyl acetate (10 mL × 2). The combined organic layers were washed with brine (10 mL), dried over anhydrous sodium sulfate, and filtered. The filtrate was concentrated, and the residue was purified by column chromatography to afford the desired product (100 mg) as a yellow solid. LC-MS (ESI, m/z) 612.1 $[M+H]^+$. $^1$HNMR (400 MHz, DMSO-$d_6$) δ 9.64 (s, 1H), 9.57 (s, 1H), 8.95 (s, 1H), 8.60 (d, *J* = 4.4 Hz, 1H), 8.48 (s, 1H), 7.90 - 7.86 (m, 1H), 7.59 (d, *J* = 7.2 Hz, 1H), 7.40 (s, 2H), 7.38 - 7.36 (m, 1H), 7.26 (d, *J* = 8.8 Hz, 1H), 7.21 - 7.19 (m, 1H), 6.71 - 6.66 (m, 1H), 6.61 - 6.57 (m, 1H), 5.29 (m, 2H), 4.32 (q, *J* = 6.8 Hz, 2H), 3.69 - 3.61 (m, 2H), 3.52 - 3.42 (m, 2H), 3.27 - 3.09 (m, 3H), 2.26 (br s, 6H), 1.47 (t, *J* = 6.8 Hz, 3H).

**Preparation Example 2**

**(E)-N-(4-((3-chloro-4-(pyridin-2-ylmethoxy)phenyl)amino)-3-cyano-7-ethoxyquinolin-6-yl)-4-(3,3-difluoroazetidin-1-yl)but-2-enamide (Compound A0902)**

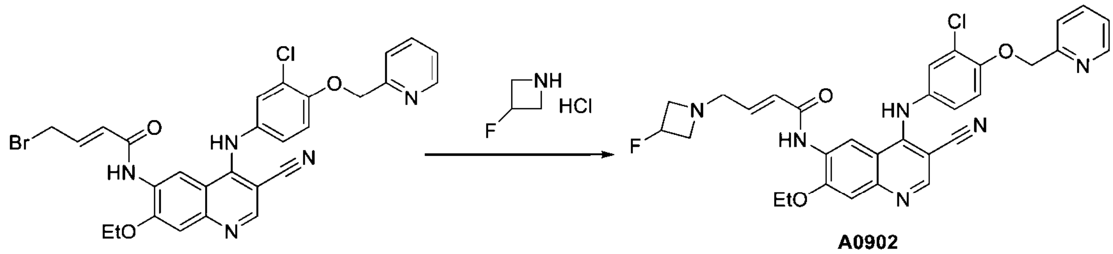

A0902

To a solution of (2E)-4-bromo-N-(4-{[3-chloro-4-(pyridin-2-ylmethoxy)phenyl]amino } -3-cyano-7-ethoxyquinolin-6-yl)but-2-enamide (100 mg, 83% purity, 0.169 mmol) in DMF (3 mL) was added potassium carbonate (70.0 mg, 0.506 mmol) and 3-fluoroazetidine hydrochloride (25.5 mg, 0.337 mmol). The reaction mixture was poured into water (10 mL) and extracted with ethyl acetate (10 mL × 2). The combined organic layers were washed with brine (10 mL), dried over anhydrous sodium sulfate, and filtered. The filtrate was concentrated, and the residue was purified by column chromatography to afford 110 mg of the desired product as a yellow solid. LC-MS (ESI, m/z) 587.2 $[M+H]^+$.

**Preparation Example 3**

**(E)-N-(4-((3-chloro-4-(pyridin-2-ylmethoxy)phenyl)amino)-3-cyano-7-ethoxyquinolin-6-yl)-4-(3,3-difluoroazetidin-1-yl)but-2-enamide (Compound A0903)**

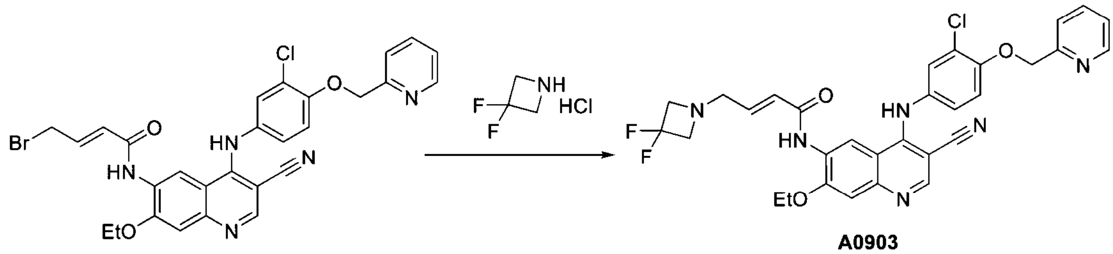

A0903

To a solution of (2E)-4-bromo-N-(4-{[3-chloro-4-(pyridin-2-ylmethoxy)phenyl]amino} -3-cyano-7-ethoxyquinolin-6-yl)but-2-enamide (100 mg, 83% purity, 0.169 mmol) in DMF (3 mL) was added potassium carbonate (70.0 mg, 0.506 mmol) and 3,3-difluoroazetidine hydrochloride (43.7 mg, 0.337 mmol). The reaction mixture was poured into water (10 mL) and extracted with ethyl acetate (10 mL × 2). The combined organic layers were washed with brine (10 mL), dried over anhydrous sodium sulfate, filtered, and concentrated. The residue was purified by column chromatography to afford 120

mg of yellow solid. LC-MS (ESI, m/z) 605.0 [M+H]+. 1HNMR (400 MHz, DMSO-d6) δ 9.61 (s, 1H), 9.52 (s, 1H), 8.95 (s, 1H), 8.60 (d, J = 4.0 Hz, 1H), 8.47 (s, 1H), 7.90 - 7.85 (m, 1H), 7.58 (d, J = 7.6 Hz, 1H), 7.39 - 7.36 (m, 3H), 7.25 (d, J = 8.8 Hz, 1H), 7.20 - 7.18 (m, 1H), 6.76 - 6.70 (m, 1H), 6.60 - 6.56 (m, 1H), 5.28 (s, 2H), 4.32 (q, J = 6.8 Hz, 2H), 3.70 - 3.63 (m, 4H), 3.41 (d, J = 4.0 Hz, 2H), 1.47 (t, J = 7.2 Hz, 3H).19F NMR (376 MHz, DMSO-$d_6$) -97.41 (s, 2F).

**Preparation Example 4**

**(E)-N-(4-((3-chloro-4-(pyridin-2-ylmethoxy)phenyl)amino)-3-cyano-7-ethoxyquinolin-6-yl)-4-(methanesulfonamido)but-2-enamide (Compound A0904)**

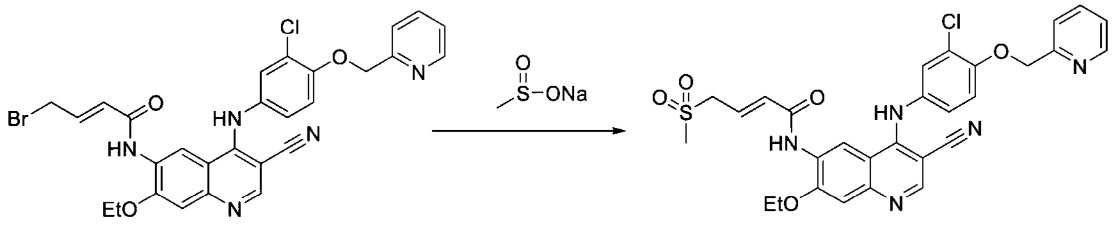

A0904

At 25°C, to a solution of (2E)-4-bromo-N-(4-((3-chloro-4-(pyridin-2-ylmethoxy)phenyl)amino)-3-cyano-7-ethoxyquinolin-6-yl)but-2-enamide (180 mg, 0.304 mmol) in dioxane (4 mL) and water (2 mL) was added sodium methanesulfinate (46.5 mg, 0.455 mmol). The resulting mixture was stirred at 100°C. After completion, the reaction mixture was cooled to 25°C, poured into water (10 mL), and extracted with ethyl acetate (10 mL × 2). The combined organic layers were washed with brine (10 mL), dried over anhydrous sodium sulfate, filtered, and concentrated. The crude product was purified by column chromatography to afford 220 mg of the desired product as a yellow solid. LC-MS (ESI, m/z) 592.0 [M+H]+. 1H NMR (400 MHz, DMSO-d6) δ 9.73 (s, 1H), 9.63 (s, 1H), 8.93 (s, 1H), 8.60 (d, J = 5.2 Hz, 1H), 8.48 (s, 1H), 7.90 - 7.85 (m, 1H), 7.58 (d, J = 8.0 Hz, 1H), 7.40 (d, J = 2.0 Hz, 2H), 7.39 - 7.36 (m, 1H), 7.27 - 7.25 (m, 1H), 7.22 - 7.19 (m, 1H), 6.76 - 6.74 (m, 2H), 5.29 (s, 2H), 4.32 (q, J = 7.2 Hz, 2H), 4.20 (d, J = 5.2 Hz, 2H), 3.02 (s, 3H), 1.47 (t, J = 7.2 Hz, 3H).

**Preparation Example 5**

**Synthesis of N-(4-((3-chloro-4-(pyridin-2-ylmethoxy)phenyl)amino)-3-cyano-7-ethoxyquinolin-6-yl)-2-((3-(dimethylamino)azetidin-1-yl)methyl)propenamide (Compound A0905)**

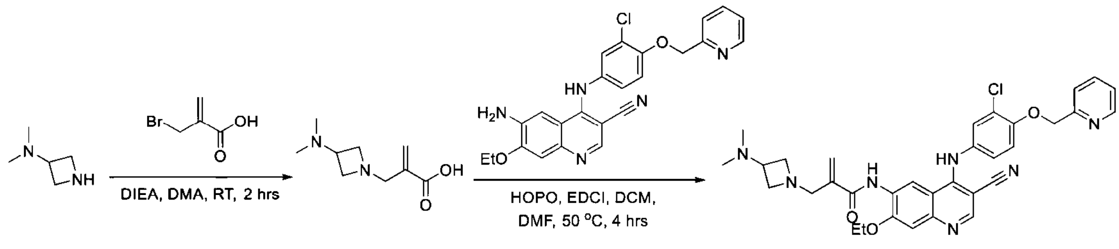


A0905

**Step 1: Synthesis of 2-((3-(dimethylamino)azetidin-1-yl)methyl)propenoic acid**

N,N-dimethylazetidin-3-amine (0.33 g, 2.00 mmol) and 2-(bromomethyl)prop-2-enoate (0.35 g, 2.00 mmol) were dissolved in N,N-dimethylacetamide (20 mL), followed by addition of DIEA (1 mL, 6.00 mmol). The reaction mixture was stirred at room temperature for 2 hours. After completion, the reaction mixture was concentrated and directly purified by reversed-phase chromatography (0-20% acetonitrile) to afford the target product as a white solid (0.20 g, 55.4% yield). LC-MS (ESI, *m/z*)185.2$[M+H]^+$; $^1$H NMR (400 MHz, DMSO-$d_6$) δ 12.06 (s, 1H), 6.30 (s, 1H), 5.97 (s, 1H), 3.99-3.84 (m, 7H), 2.54 (s, 6H).

**Step 2: Synthesis of N-(4-((3-chloro-4-(pyridin-2-ylmethoxy)phenyl)amino)-3-cyano-7-ethoxyquinolin-6-yl)-2-((3-(dimethylamino)azetidin-1-yl)methyl)propenamide**

6-Amino-4-((3-chloro-4-(pyridin-2-ylmethoxy)phenyl)amino)-7-ethoxyquinoline-3-carbonitrile (0.11 g, 0.25 mmol) and 2-((3-(dimethylamino)azetidin-1-yl)methyl)propenoic acid (0.10 g, 0.50 mmol) were dissolved in DMSO (1.5 mL), followed by addition of DIEA (0.13 mL, 0.75 mmol). HOPO (0.03 g, 0.28 mmol) and EDCI (0.05 g, 0.28 mmol) were dissolved in DCM (1.5 mL). The reaction mixture was heated to 50°C and stirred for 4 hours. After completion, the reaction mixture was concentrated, quenched with water (5 mL), and extracted with ethyl acetate (5 mL × 3). The combined organic layers were washed with brine (10 mL × 3), dried over anhydrous sodium sulfate, filtered, and concentrated. The residue was purified by preparative HPLC (0.1% FA) to afford the target compound as a white solid (121.00 mg, 79.08% yield). LC-MS (ESI, *m/z*)612.2[M+H]$^+$;$^1$H NMR (400 MHz, MeOD-$d_4$) δ 8.67 (d, *J* = 4.8 Hz, 1H), 8.62 (s, 1H), 8.12-8.08 (m, 1H) 7.84 (d, *J* = 8.0 Hz, 1H), 7.59-7.56 (m, 2H), 7.40-7.25 (m, 4H), 5.55 (d, *J* = 8.0 Hz, 2H), 5.42 (s, 2H), 4.73 (s, 1H), 4.62(S, 1H), 4.43-4.09 (m, 9H), 2.83(s, 6H) 1.61 (t, *J* = 7.2 Hz, 3H).

**Preparation Example 6**

**Synthesis of N-(4-((3-chloro-4-(pyridin-2-ylmethoxy)phenyl)amino)-3-cyano-7-ethoxyquinolin-6-yl)-2-((4-(dimethylamino)piperidin-1-yl)methyl)propenamide (Compound A0906)**

**Step 1: Synthesis of 2-((4-(dimethylamino)piperidin-1-yl)methyl)propenoic acid**

N,N-dimethylpiperidin-4-amine (0.40 g, 2.00 mmol) and 2-(bromomethyl)prop-2-enoate (0.33 g, 2.00 mmol) were dissolved in N,N-dimethylacetamide (20 mL), followed by addition of DIEA (1 mL, 6.0 mmol). The reaction mixture was stirred at room temperature for 2 hours. After completion, the reaction mixture was concentrated and directly purified by reversed-phase chromatography (0-20% acetonitrile) to afford the target product as a white oily compound (0.30 g, 56.6% yield). LC-MS (ESI,*m/z*) 213.4[M+H]$^+$; $^1$H NMR (400 MHz, MeOD-$d_1$) δ 8.38 (s, 1H), 6.27 (s, 1H), 5.76 (s, 1H), 3.69 (s, 2H), 3.58-3.52 (m, 1H), 3.45-3.30 (m, 4H), 2.83-2.81 (m, 6H), 2.30-2.20 (m, 4H).

**Step 2: Synthesis of N-(4-((3-chloro-4-(pyridin-2-ylmethoxy)phenyl)amino)-3-cyano-7-ethoxyquinolin-6-yl)-2-((4-(dimethylamino)piperidin-1-yl)methyl)propenamide**

6-Amino-4-((3-chloro-4-(pyridin-2-ylmethoxy)phenyl)amino)-7-ethoxyquinoline-3-carbonitrile (0.09 g, 0.20 mmol) and 2-((4-(dimethylamino)piperidin-1-yl)methyl)propenoic acid (0.06 g, 0.30 mmol) were dissolved in DMSO (1.0 mL), followed by addition of DIEA (0.1 mL, 0.6 mmol). 2-Hydroxypyridine N-oxide (0.02 g, 0.22 mmol) and 1-(3-dimethylaminopropyl)-3-ethylcarbodiimide hydrochloride (0.04 g, 0.22 mmol) were dissolved in DCM (1.0 mL). The reaction mixture was heated to 50°C and stirred for 4 hours. After completion, the reaction mixture was concentrated, quenched with water (5 mL), and extracted with ethyl acetate (5 mL × 3). The combined organic layers were washed with brine (10 mL × 3), dried over anhydrous sodium sulfate, and concentrated. The residue was purified by preparative HPLC (0.1% FA) to afford the target compound as a white solid (35.00 mg, 79.08% yield). LC-MS (ESI, m/z) 640.2 [M+H]+; $^1$H NMR (400 MHz, MeOD-$d_4$) δ 8.54 (d, J = 4.8 Hz, 1H), 8.51 (s, 1H), 7.99-7.94 (m, 1H), 7.72 (d, J = 8.0 Hz, 1H), 7.49 (d, J = 2.4 Hz, 1H), 7.46-7.43 (m, 1H), 7.32-7.29 (m, 1H), 7.20-7.14 (m, 4H), 5.30 (s, 2H), 5.27 (s, 1H), 5.19 (s, 1H), 4.64 (s, 1H), 4.32-4.27 (m, 2H), 4.20 (s, 1H), 4.11 (s, 2H), 3.41-3.34 (m, 1H), 3.20 (s, 1H), 2.77 (s, 6H), 2.80 (s, 1H), 2.02 (s, 2H), 1.51-1.48 (m, 5H).

**Example A: In Vitro Pharmacodynamic Evaluation of Inhibitors**

This experiment employed the CellTiter-Glo® (CTG) assay kit, a homogeneous cell viability assay that quantifies ATP to determine the viability of cultured cells. Due to its high sensitivity and simple workflow, it is currently a widely used cell viability assay. The purpose of this experiment was to evaluate the effects of six test compounds on the proliferation of a single EGFR cell line using the CTG method, with afatinib as the reference compound.

**1) Experimental Procedure**

a) All cell lines were cultured in complete growth medium at 37°C under 5% $CO_2$ conditions.

b) Cells in the logarithmic growth phase were harvested and counted using a hemocytometer. Cell viability was

assessed by trypan blue exclusion to ensure viability above 90%.

c) Cells were adjusted to the desired density with complete growth medium and seeded into 96-well plates at 3,000 cells per well in 90 μL medium.

d) The seeded 96-well plates were incubated at 37°C under 5% $CO_2$.

e) Ten-fold stock solutions of each compound were prepared, with the highest test concentration of 1 μM. Nine concentrations were generated by four-fold serial dilution. Subsequently, 10 μL of each diluted compound was transferred into the corresponding wells of the 96-well plates, with triplicate wells for each concentration.

f) The compound-treated 96-well plates were incubated for an additional 72 hours at 37°C under 5% $CO_2$, followed by CTG analysis.

g) The CTG reagent was thawed and the cell plates were equilibrated to room temperature for 30 minutes.

h) An equal volume of CTG reagent was added to each well.

i) The plates were shaken on a plate shaker for 5 minutes to induce cell lysis.

j) Plates were incubated at room temperature for 20 minutes to stabilize the luminescent signal.

k) Luminescence was measured and data were collected.

**2) Data Analysis**

Data were analyzed using GraphPad Prism 7.0 software. Nonlinear sigmoidal regression was applied to fit the dose-response curves, from which $IC_{50}$ values were calculated.

Cell viability (%) was determined according to the following equation:

$$\text{Cell viability (\%)}=(\text{Lum}_{\text{compound}}-\text{Lum}_{\text{medium control}})/(\text{Lum}_{\text{vehicle control}}-\text{Lum}_{\text{medium control}})\times 100\%$$

**3) Anti-Proliferative Effects of the Test Compounds**

| No. | Compound No. | $IC_{50}$ (nM) |
|---|---|---|
| | | Ba/F3-EGFR-G719S cell line |
| 1 | A009 (Neratinib maleate) | 2.89 |
| 2 | A0901 | 16.82 |
| 3 | A0902 | 2.53 |
| 4 | A0903 | 2.06 |
| 5 | A0904 | 2.18 |
| 6 | A0905 | 69.59 |
| 7 | A0906 | 151.60 |

The method for moisture determination of the particles or compositions of the present invention is by infrared rapid moisture analyzer (measuring temperature 105 °C). This method is a commonly used method for moisture determination known to those skilled in the art, and is widely applied to the determination of moisture content in granules, tablets, and powders. In the following, neratinib maleate refers to neratinib combined with one maleate.

**Example 1**

Neratinib maleate tablets, specification 40 mg (calculated as $C_{30}H_{29}ClN_6O_3$). The formulation is shown in Table 1:

Table 1 Formulation of Neratinib Maleate Tablets in Example 1

| Ingredient | | Unit Dose (mg) | Proportion (%) | Function |
|---|---|---|---|---|
| Neratinib maleate (anhydrous) | | 48.31 | 34.92 | Active ingredient |
| Mannitol | | 36.19 | 26.16 | Filler |
| Microcrystalline cellulose | | 11.50 | 8.31 | Filler |
| Copovidone | | 30.00 | 21.69 | Binder |
| Crospovidone XL-10 | intragranular | 3.42 | 2.47 | Disintegrant |
| | extragranular | 3.42 | 2.47 | |
| Colloidal silicon dioxide | | 2.74 | 1.98 | Glidant |
| Magnesium stearate | intragranular | 1.37 | 1.00 | Lubricant |
| | extragranular | 1.37 | 1.00 | |
| Tablet core | | 138.32 | 100.00 | / |

According to the above formulation, granulation was carried out using a dry granulator under the parameter settings shown in Table 2.

Table 2 Parameters of the Dry Granulator

| Roller Pressure bar | Feeding Speed rpm | Roller Speed rpm | Granulating Speed rpm | Sieve Aperture mm |
|---|---|---|---|---|
| 80 | 50.0 | 6.0 | 100 | $\phi$ 0.8 |

The obtained granules were mixed with the external components of the granules, namely crospovidone XL-10 and magnesium stearate. The mixture was compressed into tablets using a tablet press (10.5 mm × 5.5 mm shaped punch), with the hardness controlled at 70-140 N. Subsequently, the tablets were film-coated using a high-efficiency coating machine with a 15 w/v% aqueous suspension of a coating agent mainly composed of polyvinyl alcohol (OPADRY), so that the coating weight per tablet was 4.15 mg. The X-ray powder diffraction (XRPD) pattern of the tablets is shown in Figure 1.

During the coating process, the parameters listed in Table 3 were applied to control the coating procedure, ensuring that the maximum moisture content of the tablets during coating was maintained below 3%.

Table 3 Parameters for the Coating Process

| Parameter | Value |
|---|---|
| Inlet air temperature | 55~60°C |
| Atomization pressure | 0.10~0.30MPa |
| Peristaltic pump speed | 2.0~12.0rpm |
| Material temperature (i.e., outlet air temperature) | 35~42°C |

**Example 2**

Neratinib maleate tablets, specification 40 mg (calculated as $C_{30}H_{29}ClN_6O_3$). The formulation is shown in Table 4:

Table 4 Formulation of Neratinib Maleate Tablets in Example 2

| Ingredient | | Unit Dose (mg) | Proportion (%) | Function |
|---|---|---|---|---|
| Neratinib maleate (anhydrous) | | 48.31 | 42.26 | Active ingredient |
| Mannitol | | 36.19 | 31.66 | Filler |
| Microcrystalline cellulose | | 14.953 | 13.08 | Filler |
| Copovidone | | 9.144 | 8.00 | Binder |
| Crospovidone XL-10 | | 2.286 | 2.00 | Disintegrant |
| Colloidal silicon dioxide | | 2.29 | 2.00 | Glidant |
| Magnesium stearate | intragranular | 1.15 | 1.00 | Lubricant |
| | extragranular | 1.15 | 1.00 | |
| Tablet core | | 115.473 | 100.00 | / |

According to the above formulation, granulation was carried out using a dry granulator under the parameter settings shown in Table 5.

Table 5 Parameters of the Dry Granulator

| Roller Pressure bar | Feeding Speed rpm | Roller Speed rpm | Granulating Speed rpm | Sieve Aperture mm |
|---|---|---|---|---|
| 65 | 40.0 | 6.0 | 100 | ɸ 0.8 |

The obtained granules were then mixed with the extra-granular component magnesium stearate, and the mixture was compressed into tablets using a tablet press (10.5 mm × 5.5 mm shaped punch), with the hardness controlled at 70-140 N. Subsequently, the tablets were film-coated using a high-efficiency coating machine with a 15 w/v % aqueous suspension of a coating agent mainly composed of polyvinyl alcohol (OPADRY), so that the amount of coating per tablet was 4.15 mg. The X-ray powder diffraction (XRPD) pattern of the tablets is shown in Figure 2.

During the coating process, the parameters listed in Table 3 were applied to control the coating procedure, ensuring that the maximum moisture content of the tablets during coating was maintained below 3%.

**Example 3**

Neratinib maleate tablets, specification 40 mg (calculated as $C_{30}H_{29}ClN_6O_3$). The formulation is shown in Table 6:

Table 6 Formulation of Neratinib Maleate Tablets in Example 3

| Ingredient | | Unit Dose (mg) | Proportion (%) | Function |
|---|---|---|---|---|
| Neratinib maleate (anhydrous) | | 48.31 | 36.13 | Active ingredient |
| Mannitol | | 36.19 | 27.06 | Filler |
| Microcrystalline cellulose | | 11.50 | 8.60 | Filler |
| Copovidone | | 30.00 | 22.43 | Binder |
| Crospovidone XL-10 | | 2.25 | 1.68 | Disintegrant |
| Colloidal silicon dioxide | | 2.74 | 2.05 | Glidant |
| Magnesium stearate | intragranular | 1.37 | 1.02 | Lubricant |
| | extragranular | 1.37 | 1.02 | |
| Tablet core | | 133.73 | 100.00 | / |

According to the above formulation, granulation was carried out using a dry granulator under the parameter settings shown in Table 7.

Table 7 Parameters of the Dry Granulator

| Roller Pressure bar | Feeding Speed rpm | Roller Speed rpm | Granulating Speed rpm | Sieve Aperture mm |
|---|---|---|---|---|
| 100 | 40.0 | 6.0 | 100 | ∅ 0.8 |

The obtained granules were then mixed with the extra-granular component magnesium stearate, and the mixture was compressed into tablets using a tablet press (10.5 mm × 5.5 mm shaped punch), with the hardness controlled at 70-140 N. Subsequently, the tablets were film-coated using a high-efficiency coating machine with a 15 w/v % aqueous suspension of a coating agent mainly composed of polyvinyl alcohol (OPADRY), so that the amount of coating per tablet was 4.15 mg. The X-ray powder diffraction (XRPD) pattern of the tablets is shown in Figure 3. During the coating process, the parameters listed in Table 3 were applied to control the coating procedure, ensuring that the maximum moisture content of the tablets during coating was maintained below 3%.

**Example 4**

Neratinib maleate tablets, specification 40 mg (calculated as $C_{30}H_{29}ClN_6O_3$). The formulation is shown in Table 8:

Table 8 Formulation of Neratinib Maleate Tablets in Example 4

| Ingredient | | Unit Dose (mg) | Proportion (%) | Function |
|---|---|---|---|---|
| Neratinib maleate (anhydrous) | | 48.31 | 36.13 | Active ingredient |
| Mannitol | | 36.19 | 27.06 | Filler |
| Microcrystalline cellulose | | 11.50 | 8.60 | Filler |
| Copovidone | | 30.00 | 22.43 | Binder |
| Crospovidone XL-10 | | 2.25 | 1.68 | Disintegrant |
| Gel silicon dioxide | | 2.74 | 2.05 | Glidant |
| Magnesium stearate | intragranular | 1.37 | 1.02 | Lubricant |
| | extragranular | 1.37 | 1.02 | |
| Tablet core | | 133.73 | 100.00 | / |

According to the above formulation, granulation was carried out using a dry granulator under the parameter settings shown in Table 9.

Table 9 Parameters of the Dry Granulator

| Roller Pressure bar | Feeding Speed rpm | Roller Speed rpm | Granulating Speed rpm | Sieve Aperture mm |
|---|---|---|---|---|
| 100 | 40.0 | 6.0 | 100 | f 0.8 |

The obtained granules were then mixed with the extra-granular component magnesium stearate, and the mixture was compressed into tablets using a tablet press (10.5 mm × 5.5 mm shaped punch), with the hardness controlled at 70-140 N. Subsequently, the tablets were film-coated using a high-efficiency coating machine with a 15 w/v % aqueous suspension of a coating agent mainly composed of polyvinyl alcohol (OPADRY), so that the amount of coating per tablet was 4.15 mg. The X-ray powder diffraction (XRPD) pattern of the tablets is shown in Figure 6.

During the coating process, the parameters listed in Table 3 were applied to control the coating procedure, ensuring that the maximum moisture content of the tablets during coating was maintained below 3%.

**Example 5**

The formulation and process described in Example 4 were scaled up for production in a manufacturing facility, with the batch size increased from several hundred tablets at laboratory scale to 100,000 tablets, and three consecutive batches were produced, designated as Scale-up Batch 1, Scale-up Batch 2, and Scale-up Batch 3. The formulation is shown in Table 10.

Table 10 Formulation of Neratinib Maleate Tablets in Example 5

| Ingredient | | Unit Dose (mg) | Proportion (%) | Batch formulation (g) | Function |
|---|---|---|---|---|---|
| Neratinib maleate (anhydrous) | | 48.31 | 36.13 | 4831 | Active ingredient |
| Mannitol | | 36.19 | 27.06 | 3619 | Filler |
| Microcrystalline cellulose | | 11.50 | 8.60 | 1150 | Filler |
| Copovidone | | 30.00 | 22.43 | 3000 | Binder |
| Crospovidone XL-10 | | 2.25 | 1.68 | 225 | Disintegrant |
| Gel silicon dioxide | | 2.74 | 2.05 | 274 | Glidant |
| Magnesium stearate | intragranular | 1.37 | 1.02 | 137 | Lubricant |
| | extragranular | 1.37 | 1.02 | 137 | |
| Tablet core | | 133.73 | 100.00 | 13.37 | / |

According to the above formulation, granulation was carried out using a dry granulator under the parameter settings shown in Table 11.

Table 11 Parameters of the Dry Granulator

| Roller Pressure bar | Feeding Speed rpm | Roller Speed rpm | Granulating Speed rpm | Sieve Aperture mm |
|---|---|---|---|---|
| 100 | 40.0 | 6.0 | 100 | ɸ 0.8 |

The obtained granules were then mixed with the extra-granular component magnesium stearate, and the mixture was compressed into tablets using a tablet press (10.5 mm × 5.5 mm shaped punch), with the hardness controlled at 70-140 N. Subsequently, the tablets were film-coated using a high-efficiency coating machine with a 15 w/v % aqueous suspension of a coating agent mainly composed of polyvinyl alcohol (OPADRY), so that the amount of coating per tablet was 4.15 mg. The X-ray powder diffraction (XRPD) pattern of the tablets is shown in Figure 7-9.

During the coating process, the parameters listed in Table 3 were applied to control the coating procedure, ensuring that the maximum moisture content of the tablets during coating was maintained below 3%.

**Example of Effect:**

The neratinib maleate tablets of Examples 1, 2, and 3 were scaled up to pilot production according to their respective formulations. The scale-up was performed for Examples 1, 2, and 3. The three batches of neratinib maleate tablets were compared with the reference product. The reference product selected was the neratinib maleate original drug (coated tablet) from Puma Biotechnology.

**Effect Example 1:** Related Substances Test

The related substances of the first batch of Examples 1, 2, 3, and the reference product were tested. The test results are shown in Table 12. The two batches of neratinib maleate tablets obtained in Example 3 showed lower degradation impurity (impurity 1) and total impurities than those in the reference product.

Table 12 Related Substances Test Results

| Batch No. | Example 1 | Example 2 | Example 3 | Reference product |
|---|---|---|---|---|
| Impurity 1 (degradation impurity) | 0.12% | 0.09% | 0.23% | 0.98% |
| Impurity 2 | 0.05% | 0.05% | 0.02% | 0.02% |
| Total Impurities | 0.30% | 0.26% | 0.28% | 1.17% |

**Effect Example 2:** Results of Moisture and Crystalline Form Testing

The moisture content and crystalline form of the first batches of the preparations of Examples 1, 2, 3, and the

reference product were tested. The results are shown in Table 13.

Table 13 Results of Moisture and Crystalline Form Testing

| Batch No. | Example 1 | Example 2 | Example 3 | Reference product |
|---|---|---|---|---|
| Moisture | 2.4% | 1.9% | 2.8% | 3.0% |
| Crystalline Form | Anhydrous form, see Fig. 1 | Anhydrous form, see Fig. 2 | Anhydrous form, see Fig.3 | Hydrated form, see Fig. 4 |

**Effect Example 3:** Stability Study

Stability samples of the first batches of Examples 1, 2, 3, and the reference product were taken to investigate the increase of degradation impurities. The results are shown in Table 14.

Table 14 Increase of Degradation Impurities in the Stability Study of Neratinib Maleate

| 40°C, RH 75% Accelerated | Example 1 | Example 2 | Reference product |
|---|---|---|---|
| 0 month | 0.12% | 0.09% | 0.98% |
| 1 month | 0.60% | 0.57% | 1.31% |
| 2 month | 0.91% | 0.81% | 1.50% |
| 3 month | 1.11% | 0.99% | 1.61% |
| 6 month | 1.34% | 1.25% | 1.77% |

As shown in the above table, after 6 months under accelerated conditions, the content of degradation impurities in Examples 1, 2, and 3 is lower than that of the reference product and meets the quality standard (≤1.5%), whereas the reference product has already exceeded the limit. This indicates that the tablets of the present invention have good stability.

**Effect Example 4:** Dissolution Profile Evaluation

The dissolution profiles were determined based on the Dissolution and Release Test Method (General Rule 0931, Method II, Chinese Pharmacopoeia 2020, Part IV).

The dissolution conditions were as follows: paddle method at pH 1.2, 500 mL, 50 rpm; and paddle method at pH 3.0, 900 mL, 75 rpm. Samples were taken at various time points from 5 to 60 minutes. An appropriate volume of the dissolution medium was withdrawn and filtered, discarding at least the first 2 mL, and the filtrate was collected for analysis. An appropriate amount of neratinib maleate reference standard was accurately weighed, dissolved in the dissolution medium using ultrasound, and diluted to prepare a solution containing approximately 44 μg of neratinib (calculated as $C_{30}H_{29}ClN_6O_3$) per mL, which was used as the reference solution. High-performance liquid chromatography (HPLC, General Rule 0512, Chinese Pharmacopoeia 2020, Part IV) was employed, using an octadecylsilyl bonded silica column as the stationary phase (recommended: Waters XBridge C18, 4.6 mm × 150 mm, 5 μm, or a column of equivalent performance). The mobile phase was 0.1% trifluoroacetic acid solution-methanol (67:33). Detection was performed at 266 nm, column temperature at 40°C, flow rate 1.5 mL/min, run time 10 minutes, and injection volume 10 μL. The sample solution and reference solution were precisely measured and injected into the HPLC system, and chromatograms were recorded. The amount of neratinib dissolved at each time point per tablet was calculated based on the peak area using the external standard method. The similarity factor ($f_2$) was used to compare the dissolution profile with that of the first batch of the reference product.

$$f_2 = 50\log\left[\frac{100}{\sqrt{1+\frac{\sum_{i=1}^{n}(R_t-T_t)^2}{n}}}\right]$$

In the above formula, *n* represents the number of time points; $R_t$ is the mean percentage of drug released from the reference product at time *t;* $T_t$ is the mean percentage of drug released from the test preparation at time *t*. An $f_2$ value ≥ 50 indicates that the dissolution profiles of the test preparation and the reference product are considered similar. Detailed dissolution data are shown in Tables 15-16.

Table 15 Dissolution Data of Each Example in pH 1.2 Medium (50 rpm-500 mL)

| Time point /min | Reference product Batch 1 | Example 1 | Example 2 | Example 3 |
|---|---|---|---|---|
| 5 | 12 | 19 | 15 | 14 |
| 10 | 27 | 41 | 28 | 32 |
| 15 | 45 | 59 | 39 | 47 |
| 20 | 64 | 72 | 52 | 60 |
| 30 | 90 | 87 | 69 | 78 |
| 45 | 100 | 93 | 81 | 94 |
| 60 | 100 | 95 | 90 | 99 |
| Similarity factor $f_2$ | N/A | 50 | 54 | 60 |

Table 16 Dissolution data of each Example in pH 3.0 medium (75 rpm-900 mL)

| Time point /min | Reference product Batch 1 | Example 1 | Example 2 | Example 3 |
|---|---|---|---|---|
| 5 | 6 | 7 | 9 | 4 |
| 10 | 22 | 20 | 24 | 15 |
| 15 | 35 | 31 | 37 | 28 |
| 20 | 46 | 41 | 49 | 39 |
| 30 | 60 | 56 | 66 | 58 |
| 45 | 68 | 73 | 78 | 77 |
| 60 | 77 | 83 | 90 | 88 |
| 90 | 88 | 96 | 98 | 98 |
| 120 | 90 | N/A | N/A | N/A |
| Similarity factor $f_2$ | N/A | 65 | 58 | 58 |

All products of the Examples in the present invention showed similar dissolution behavior to the reference product in discriminatory dissolution testing (similarity factor $f_2$ ≥ 50), indicating equivalent in vivo bioactivity.

**Effect Example 5:**

The total mixed granules from the preparation process of the neratinib maleate tablets of Examples 1, 2, and 3 were evaluated, as shown in Tables 17, 18, and 19. The dissolution profiles of Example 1, Example 2, and the first batch of the reference product are shown in Figure 5.

A+2.2S test method: 10 tablets of the test product were taken. The absolute value of the difference between the labeled content (100) and the measured mean is defined as A, and S is the standard deviation. A+2.2S ≤ 15.0 indicates that

the content uniformity of the product meets the requirement.

Table 17 Evaluation of total mixed granules blend uniformity

| Total mixed granules | | Example 1 | Example 2 | Example 3 |
|---|---|---|---|---|
| Blend uniformity | Average content | 23.93% | 35.04% | 29.91% |
| | RSD | 0.50% | 0.37% | 0.25% |
| Carr's index | | 13.6% | 17.1% | 16.9% |

Table 18 Evaluation of tablet weight variation and hardness

| | Example 1 | Example 2 | Example 3 |
|---|---|---|---|
| Tablet weight variation | < 5% | < 5% | < 5% |

Table 19 Evaluation of coated tablet parameters

| Coated tablets | | Example 1 | Example 2 | Example 3 |
|---|---|---|---|---|
| Content uniformity | Average content | 100.6% | 101.8% | 100.2% |
| | A+2.2S | 6.0 | 4.2 | 0.25% |

The above results indicate that the total mixed granules obtained after the dry granulation process of the present invention have uniform content, good flowability, and do not affect the compressibility or flow properties of the material during tableting.

**Effect Example 6:**

The tablets prepared in Example 4 were evaluated, and the results are shown in Table 20.

Table 20 Examination Results of Example 4

| Test Item | Content | Related Substances | Tablet Weight Variation | Content Uniformity | Moisture |
|---|---|---|---|---|---|
| Example 4 | 98.6% | Impurity 1:0.15%<br>Impurity 2:0.05%<br>Total impurities: 0.23% | < 5% | 2.1% | 2.2% |

The above results indicate that the tablets prepared in Example 4 have performance comparable to those of Examples 1-3.

**Effect Example 7:**

Three batches produced in the scale-up of Example 5 were evaluated. During the production process, monitoring was performed. Granule size results are shown in Table 21, blend uniformity results in Table 22, and tablet weight variation and hardness results in Table 23.

Table 21 Granule Size Results

| **Item** | **Scale-up Batch 1** | **Scale-up Batch 2** | **Scale-up Batch 3** |
|---|---|---|---|
| 30-mesh sieve | 5.12% | 5.13% | 3.80% |
| 40-mesh sieve | 43.25% | 38.05% | 40.29% |
| 60-mesh sieve | 16.27% | 17.26% | 17.14% |
| 80-mesh sieve | 5.63% | 6.65% | 6.67% |
| 150-mesh sieve | 16.27% | 16.91% | 16.74% |

(continued)

| Item | Scale-up Batch 1 | Scale-up Batch 2 | Scale-up Batch 3 |
|---|---|---|---|
| 200-mesh sieve | 11.06% | 12.75% | 10.67% |
| Residue | 2.29% | 3.26% | 4.67% |

Table 22 Blend Uniformity Results

| Batch No. | | Scale-up Batch 1 | Scale-up Batch 2 | Scale-up Batch 3 |
|---|---|---|---|---|
| Blend uniformity | Mean | 30.0% | 29.8% | 30.1% |
| | RSD (≤ 5%) | 0.56% | 0.46% | 0.64% |

Table 23 Tablet Weight Variation and Hardness Results

| Test Item | Compression Stage | Scale-up Batch 1 | Scale-up Batch 2 | Scale-up Batch 3 |
|---|---|---|---|---|
| Weight Variation | Early stage | <5 % | <5% | <5% |
| | Mid stage | <5% | <5 % | <5% |
| | Late stage | <5 % | <5 % | <5% |
| | Early stage | 73 ~ 96 | 78 ~ 103 | 72~104 |
| Hardness (70-140 N) | Mid stage | 81 ~ 99 | 83 ~ 102 | 82~101 |
| | Late stage | 81 ~ 109 | 83 ~ 105 | 87~107 |

The above results indicate that for the three scale-up batches produced according to the present invention, the granule size distribution during production was uniform with no significant differences among batches. Blend uniformity met the requirements, indicating that the material was well mixed and suitable for subsequent tableting. During tableting, tablet weight variation and hardness met the requirements, indicating good compressibility of the material.

**Effect Example 8:**

The content, related substances, and moisture of the three scale-up batches produced in Example 5 were tested. The results are shown in Table 24.

Table 24 Content, Related Substances, and Moisture Results

| Batch No. | | Scale-up Batch 1 | Scale-up Batch 2 | Scale-up Batch 3 |
|---|---|---|---|---|
| Related Substances | Impurity 1 | 0.19% | 0.15% | 0.17% |
| | Impurity 2 | 0.05% | 0.05% | 0.05% |
| | Total Impurities | 0.24% | 0.20% | 0.22% |
| Moisture | | 2.20% | 2.60% | 2.60% |
| Content | | 100.10% | 98.80% | 100.50% |

The above results indicate that the related substances, moisture, and content of the three batches produced according to the present invention all meet the requirements. There are no significant differences among the three batches, indicating that the products produced in scale-up according to the present invention are uniform and qualified.

**Effect Example 9**

Stability studies were conducted on the three scale-up batches produced in Example 5. The study conditions were as follows: accelerated (40 °C ± 2 °C, relative humidity 75% RH ± 5% RH) and long-term (25 °C ± 2 °C, relative humidity 60% RH ± 5% RH). Sampling time points for the accelerated study were at 1, 2, 3, and 6 months; for the long-term study, sampling time points were at 3, 6, 9, and 12 months. The evaluated parameters included related substances, content, moisture, crystal form, and dissolution profile. The reference product was the Puma Biotechnology neratinib

maleate coated tablet.

The X-ray powder diffraction (XRPD) patterns of the three scale-up batches after 6 months of accelerated testing are shown in Figures 10-12, and the XRPD patterns after 12 months of long-term testing are shown in Figures 13-15; no changes in crystal form were observed.

Dissolution testing was conducted on the three scale-up batches directly after production, before accelerated and long-term testing. The dissolution profiles are shown in Figure 16. Compared with the second batch of the reference product, the dissolution curves of the three batches were similar, indicating that the products produced according to this specification have comparable quality to the reference.

The dissolution profiles of the three scale-up batches after 6 months of accelerated testing are shown in Figure 17, and after 12 months of long-term testing in Figure 18, with simultaneous comparison to the second batch of the reference product. The dissolution characteristics of all three batches remained unchanged and were similar to the reference product.

The stability results of related substances, content, and moisture for the three scale-up batches under accelerated and long-term conditions are shown in Tables 25-30, with simultaneous comparison to the stability results of the second batch of the reference product (Tables 31-32).

Table 25 Accelerated Stability Results of Scale-up Batch 1

| Test Item | | 1 Month | 2 Month | 3 Month | 6 Month |
|---|---|---|---|---|---|
| Related Substances | Impurity 1 | 0.60% | 0.81% | 1.04% | 1.33% |
| | Impurity 2 | 0.05% | 0.05% | 0.05% | 0.05% |
| | Total Impurities | 0.65% | 0.86% | 1.09% | 1.38% |
| Content | | 99.10% | 96.40% | 97.30% | 96.60% |
| Moisture | | 2.30% | 2.10% | 2.50% | 2.50% |

Table 26 Accelerated Stability Results of Scale-up Batch 2

| Test Item | | 1 Month | 2 Month | 3 Month | 6 Month |
|---|---|---|---|---|---|
| Related Substances | Impurity 1 | 0.55% | 0.69% | 0.86% | 1.2% |
| | Impurity 2 | 0.05% | 0.05% | 0.05% | 0.06% |
| | Total Impurities | 0.60% | 0.74% | 0.91% | 1.26% |
| Content | | 99.3% | 97.5% | 96.3% | 96.9% |
| Moisture | | 2.3% | 1.9% | 2.5% | 2.1% |

Table 27 Accelerated Stability Results of Scale-up Batch 3

| Test Item | | 1 Month | 2 Month | 3 Month | 6 Month |
|---|---|---|---|---|---|
| Related Substances | Impurity 1 | 0.55% | 0.75% | 0.94% | 1.3% |
| | Impurity 2 | 0.05% | 0.06% | 0.06% | 0.06% |
| | Total Impurities | 0.60% | 0.81% | 1.00% | 1.36% |
| Content | | 100.7% | 99.3% | 97.7% | 98.0% |
| Moisture | | 2.1% | 1.9% | 2.4% | 2.0% |

Table 28 Long-term Stability Results of Scale-up Batch 1

| Test Item | | 3 Month | 6 Month | 9 Month | 12 Month |
|---|---|---|---|---|---|
| Related Substances | Impurity 1 | 0.38% | 0.47% | 0.57% | 0.66% |
| | Impurity 2 | 0.05% | 0.06% | 0.07% | 0.08% |
| | Total Impurities | 0.43% | 0.53% | 0.64% | 0.74% |
| Content | | 97.7% | 97.0% | 95.8% | 96.7% |

(continued)

| Test Item | | 3 Month | 6 Month | 9 Month | 12 Month |
|---|---|---|---|---|---|
| Moisture | | 2.5% | 2.2% | 2.0% | 2.0% |

Table 29 Long-term Stability Results of Scale-up Batch 2

| Test Item | | 3 Month | 6 Month | 9 Month | 12 Month |
|---|---|---|---|---|---|
| Related Substances | Impurity 1 | 0.32% | 0.40% | 0.49% | 0.56% |
| | Impurity 2 | 0.05% | 0.06% | 0.07% | 0.08% |
| | Total Impurities | 0.37% | 0.46% | 0.56% | 0.64% |
| Content | | 97.1% | 97.3 | 96.1% | 97.4% |
| Moisture | | 2.5% | 2.1% | 2.0% | 1.9% |

Table 30 Long-term Stability Results of Scale-up Batch 3

| Test Item | | 3 Month | 6 Month | 9 Month | 12 Month |
|---|---|---|---|---|---|
| Related Substances | Impurity 1 | 0.34% | 0.44% | 0.52% | 0.61% |
| | Impurity 2 | 0.05% | 0.06% | 0.07% | 0.08% |
| | Total Impurities | 0.39% | 0.50% | 0.59% | 0.69% |
| Content | | 98.3% | 98.1% | 98.1% | 98.6% |
| Moisture | | 2.4% | 2.6% | 2.1% | 2.0% |

Table 31 Accelerated Stability Results of Reference Product

| Test Item | | 3 Month | 6 Month |
|---|---|---|---|
| Related Substances | Impurity 1 | 1.28% | 1.7% |
| | Impurity 2 | 0.05% | 0.04% |
| | Total Impurities | 1.33% | 1.74% |
| Content | | 100.2% | 98.5% |
| Moisture | | 3.2% | 3.3% |

Table 32 Long-term Stability Results of Reference Product

| Test Item | | 3 Month | 6 Month |
|---|---|---|---|
| Related Substances | Impurity 1 | 0.75% | 0.76% |
| | Impurity 2 | 0.05% | 0.05% |
| | Total Impurities | 0.80% | 0.81% |
| Content | | 101.1% | 98.1% |
| Moisture | | 3.0% | 3.2% |

From the above results, it can be seen that the three batches of scale-up production according to the present invention exhibit good stability, minimal batch-to-batch variation, and uniform quality. Compared with the reference product, the impurities are lower.

**For Comparative Examples 1-5 and Effect Comparative Examples 1-5:**

The type or amount of binder in Table 33 was used to replace the copovidone or its amount in Example 1, while all other components and processes remained the same as in Example 1. The resulting products were tested for dissolution

according to the method of Effect Example 4. The reference product was the third batch of Puma Biotechnology neratinib maleate (film-coated tablet).

Table 33 Binder Screening Types and Amounts

| No. | Binder Type | Amount | Note |
|---|---|---|---|
| 1 | Copovidone S630 | 3.0% | Dissolution see Tables 17-18 |
| 2 | Hydroxypropyl cellulose HPC-L | 15% | Dissolution see Tables 17-18 |
| 3 | Povidone K25 | 8% | Dissolution see Tables 17-18 |
| 4 | Hydroxypropyl cellulose | 8% | Dissolution see Tables 17-18 |
| 5 | Copovidone (S-630) | 6.5% | Dissolution see Tables 17-18 |
| 6 | Copovidone (S-630) | 8% | See Example 2 |
| 7 | Copovidone (S-630) | 22% | See Example 3 |

Table 34 Dissolution Results in pH 1.2 Medium (50 rpm-500 mL)

| Time point /min | Reference Product 3rd Batch | 1 | 2 | 3 | 4 | 5 |
|---|---|---|---|---|---|---|
| 5 | 14 | 12 | 9 | 13 | 56 | 13 |
| 10 | 28 | 22 | 22 | 24 | 76 | 27 |
| 15 | 43 | 30 | 35 | 35 | 81 | 42 |
| 20 | 60 | 38 | 48 | 45 | 84 | 51 |
| 30 | 85 | 52 | 69 | 59 | 87 | 65 |
| 45 | 98 | 68 | 88 | 72 | 90 | 75 |
| 60 | 98 | 78 | 94 | 80 | 93 | 81 |
| f2 value | / | 36 | 49 | 43 | 23 | 50 |

Table 35 Dissolution Results in pH 3.0 Medium (70 rpm-900 mL)

| Time point /min | Reference Product 3rd Batch | 1 | 2 | 3 | 4 | 5 |
|---|---|---|---|---|---|---|
| 5 | 6 | 13 | 15 | 14 | 87 | 55 |
| 10 | 22 | 32 | 58 | 31 | 93 | 93 |
| 15 | 35 | 49 | 72 | 51 | 94 | 99 |
| 20 | 46 | 67 | 82 | 69 | 94 | 100 |
| 30 | 60 | 95 | 89 | 90 | 95 | 99 |
| 45 | 68 | 92 | 94 | 94 | 95 | 98 |
| 60 | 77 | 99 | 96 | 93 | 95 | 98 |
| 90 | 88 | 99 | 97 | / | / | 100 |
| 120 | 90 | 99 | 97 | / | / | / |
| f2 value | / | 32 | 24 | 32 | 10 | 12 |

From the screening results, it can be seen that the samples prepared with Copovidone S630 (3.0% or 6.5%), hydroxypropyl cellulose HPC-L (15%), Povidone K25 (8%), or hydroxypropyl cellulose (8%) exhibited dissolution profiles not similar to the reference product, indicating a high risk of non-bioequivalence with the original product.

**Biological Examples**

(A009 refers to neratinib maleate, CVL218 refers to the compound disclosed in CN103242273B

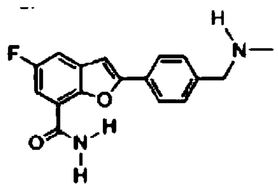

,

and CVL237 refers to the compound

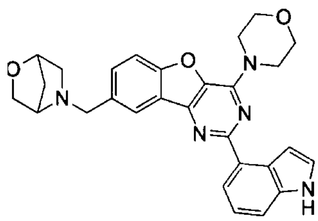

).

1. Cholangiocarcinoma PDX Model LD1-0060-200791 in Vitro Antiproliferation Assay

Tumor tissues were surgically excised from the subcutaneous tumors of tumor-bearing mice and immersed in HBSS buffer. Non-tumor and necrotic tissues were removed in a biosafety cabinet, and the tissues were cut into small pieces of 1-3 $mm^3$. Tumor pieces were digested with collagenase at 37°C for 1-2 hours. Single-cell suspensions were collected through a mesh, and centrifuged at 1200 rpm for 3 minutes to remove the supernatant. Cells were resuspended in serum-free medium, counted, and adjusted to a concentration of $1.11 \times 10^5$/mL. High-concentration drug stocks were diluted 1000× with BIO-MPM-1 medium to prepare 20× intermediate concentrations. Each well of a 96-well plate received 7.5 µL of the corresponding 20× drug solution or BIO-MPM-1 containing 2% DMSO (final DMSO concentration 0.2%). Then 135 µL of cell suspension was added per well. Cells were cultured continuously for 6 days at 37°C. Afterwards, 50 µL of CTG reagent was added per well. After mixing, 80 µL of the solution was measured in a multifunctional microplate reader for ATP content. Signals from each well were recorded to generate inhibition curves and calculate parameters such as IC50. The tumor cell proliferation inhibition rate for each drug concentration was calculated using Excel according to the formula:

$$\text{Inhibition (\%)} = ((\text{VControl-VBlank})-(\text{VDrug-VBlank}))/(\text{VControl-VBlank})\times 100\%$$

Inhibition curves were plotted using Xlfit (IDBS) to determine parameters including maximum inhibition, minimum inhibition, absolute IC50, relative IC50, and slope.

Proliferation rate (fold increase) was calculated as:

$$\text{Proliferation rate} = (V_{day6}\ \text{Control-}V_{day6}\ \text{Blank})/(V_{day0}\ \text{Control-}V_{day0}\ \text{Blank})$$

The concentration ranges are shown in Table 36.

Table 36

| **Compound** | Concentration Range |
|---|---|
| CVL218 | 0.23~30 µM |
| CVL237 | 0.23~15 µM |
| A009 | 0.23~15 µM |
| CVL218+A009 | 0.05~15 µM |
| CVL237+A009 | 0.05~15 µM |

In vitro antiproliferation assay data are shown in Table 37.

Table 37

| Compound | Minimum Inhibition Rate (%) | Maximum Inhibition Rate (%) | Absolute IC50 (μM) | Relative IC50 (μM) | Slope |
|---|---|---|---|---|---|
| CVL218 | 6.4 | 110.0 | 5.77 | 7.08 | 1.57 |
| CVL237 | -10.0 | 99.5 | 3.48 | 3.41 | 9.51 |
| A009 | -10.0 | 99.2 | 3.31 | 3.26 | 13.13 |
| CVL218+A009 | -10.0 | 99.7 | 3.20 | 3.15 | 12.13 |
| CVL237+A009 | -0.4 | 99.8 | 1.37 | 1.37 | 3.32 |

2. In vitro antiproliferation assay in Ba/F3 engineered cells harboring EGFR-G719X/E709X mutations.

This experiment employed the CellTiter-Glo (CTG) kit, a homogeneous cell viability assay that quantifies ATP to determine the viability of cultured cells. Owing to its high sensitivity and simple workflow, it is a mainstream kit for cell viability testing. The objective was to evaluate by the CTG method the effects of one test compound on the proliferation of five EGFR cell lines, with afatinib as the reference compound.

Procedure:

a) All cell lines were cultured in complete medium at 37 °C with 5% $CO_2$.
b) Cells in the logarithmic growth phase were harvested and counted with a hemocytometer. Cell viability was assessed by the trypan blue exclusion method to ensure viability >90%.
c) Cell density was adjusted with complete medium, and 90 μL per well (3,000 cells/well) was seeded into 96-well plates.
d) The 96-well plates were incubated at 37 °C with 5% $CO_2$.
e) Ten-fold stock drug solutions were prepared; the highest test concentration was 10 μM, with 9 concentrations in a 3.16-fold serial dilution. Then 10 μL of each serially diluted compound was transferred to the corresponding wells; three replicate wells were set for each concentration.
f) After dosing, plates were incubated at 37 °C with 5% $CO_2$ for 72 h, followed by CTG analysis.
g) Thaw the CTG reagent and equilibrate the plates to room temperature for 30 min.
h) Add an equal volume of CTG solution to each well.
i) Shake on an orbital shaker for 5 min to lyse cells.
j) Let the plates stand at room temperature for 20 min to stabilize the luminescent signal.
k) Read luminescence and collect data.

Data analysis:

Data were analyzed with GraphPad Prism 7.0. Nonlinear S-curve regression was used to fit dose-response curves and calculate $IC_{50}$ values.

$$\text{Cell viability (\%)} = (\text{Lum}_{\text{test}} - \text{Lum}_{\text{medium control}}) / (\text{Lum}_{\text{vehicle control}} - \text{Lum}_{\text{medium control}}) \times 100\%.$$

Antiproliferative activity of the test compound is shown in the table below.

(The cell lines in Table 38 below are all rare mutation types of non-small cell lung cancer.)

Table 38

| **No.** | **Cell line** | **IC50 (nM)** | |
|---|---|---|---|
| | | A009 | Afatinib |
| 1 | Ba/F3-EGFR-G719S | 2.89 | <1 |
| 2 | Ba/F3-EGFR-T263P-G719S | 5.1 | 1.46 |
| 3 | Ba/F3-EGFR-L747S-G719A | 9.24 | 3.49 |
| 4 | Ba/F3-EGFR-E709K-G719A | 4.91 | 2.1 |
| 5 | Ba/F3-EGFR-E709A | 10.34 | 2.25 |

3. In vivo antitumor efficacy study in Ba/F3 engineered cell EGFR-G719S xenograft model

BalF3-EGFR-G719S cells were revived and cultured in vitro to obtain $8\times10^7$ cells. Mice were shaved at the inoculation site, disinfected with povidone-iodine swabs, and subcutaneously inoculated with 0.1 mL of cell suspension ($2\times10^6$ cells/site) into the right shoulder using a 1 mL syringe. When the mean tumor volume reached approximately 100 $mm^3$, mice were randomized into 5 groups (n=8 per group) according to tumor volume and body weight: vehicle control, A009 at 20, 40, and 80 mg/kg, and afatinib at 40 mg/kg. From inoculation until randomization, body weight and tumor volume were measured once weekly. After randomization, body weight and tumor volume were measured three times weekly. Tumor length and width were measured using calipers, and tumor volume (TV) was calculated using the formula:

$$TV = 1/2 \times a \times b^2,$$

where *a* is tumor length and *b* is tumor width.

Antitumor activity was evaluated using the following indices:

$$\text{Relative tumor growth rate } (\%\Delta T/C) = (\text{mean}(T)-\text{mean}(T0)) / (\text{mean}(C)-\text{mean}(C0)) \times 100\%$$

Tumor growth inhibition rate (TGI%) = ((mean(C)-mean(C0)) - (mean(T)-mean(T0))) / (mean(C) -mean(C0)) $\times$ 100%

where T = tumor volume of treatment group, T0 = initial tumor volume of treatment group, C = tumor volume of control group, C0 = initial tumor volume of control group.

Body weight and tumor volume data were expressed as Mean $\pm$ SEM. Statistical analyses were performed using GraphPad Prism 6. For pairwise comparisons, T-test was applied; for comparisons among three or more groups, two-way ANOVA followed by Bonferroni's test was used. A value of $P < 0.05$ was considered statistically significant.

The changes in body weight and tumor volume of each group are shown in Figures 19 and 20. A009 inhibited Ba/F3-EGFR-G719S tumor growth in a dose-dependent manner. At medium and high doses, tumor regression was observed, suggesting potential efficacy in treating NSCLC harboring rare G719X mutations. At equivalent doses, A009 exhibited comparable antitumor activity to the positive control afatinib, but with overall better tolerability.

In Figures 19 and 20, QD indicates once daily dosing, and Q2D indicates once every two days.

Although the above provides specific embodiments of the present invention, those skilled in the art will understand that these are merely illustrative examples. Various modifications or alterations may be made without departing from the spirit and scope of the invention. The protection scope of the present invention is defined by the appended claims.

## Claims

1. A compound of formula I:

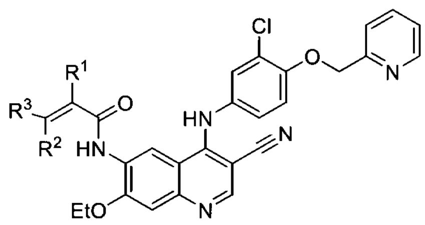

I

,

wherein:

$R^1$, $R^2$, and $R^3$ are each independently H or a $C_{1-6}$ alkyl substituted with one or more $R^{1-1}$;
each $R^{1-1}$ is independently a 4-6 membered heterocycloalkyl in which the heteroatoms are selected from one, two, or three of N, O, and S, with one, two, or three heteroatoms, a 4-6 membered heterocycloalkyl substituted with one or more $R^{1-a}$ in which the heteroatoms are selected from one, two, or three of N, O, and S, with one, two, or three heteroatoms, or $-SO_2-C_{1-6}$ alkyl;
each $R^{1-a}$ is independently halogen or $-N(C_{1-6}\ alkyl)_2$.

**2.** The compound as shown in Formula I according to claim 1, **characterized in that** it satisfies one or more of the following conditions:

(1) each $R^{1-1}$ is independently a 4-6 membered heterocycloalkyl substituted with one or more $R^{1-a}$ in which the heteroatoms are selected from one, two, or three of N, O, and S, with one, two, or three heteroatoms, or $-SO_2-C_{1-6}$ alkyl.;

(2) among $R^1$, $R^2$, $R^3$, $R^{1-1}$, and $R^{1-a}$, the $C_{1-6}$ alkyl in "$C_{1-6}$ alkyl substituted with one or more $R^{1-1}$," "$-SO_2-C_{1-6}$ alkyl," and "$-N(C_{1-6}$ alkyl$)_2$" are each independently methyl, ethyl, n-propyl, isopropyl, n-butyl, isobutyl, or tert-butyl, preferably methyl;

(3) among $R^{1-1}$, the "4-6 membered heterocycloalkyl in which the heteroatoms are selected from one, two, or three of N, O, and S, with one, two, or three heteroatoms" in the "4-6 membered heterocycloalkyl in which the heteroatoms are selected from one, two, or three of N, O, and S, with one, two, or three heteroatoms" and in the "4-6 membered heterocycloalkyl substituted with one or more $R^{1-a}$ in which the heteroatoms are selected from one, two, or three of N, O, and S, with one, two, or three heteroatoms" are each independently a 4-6 membered heterocycloalkyl containing one N heteroatom, preferably

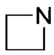 or 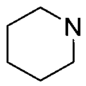 ;

(4) among $R^{1-a}$, the halogen is fluorine, chlorine, bromine, or iodine, preferably fluorine.

**3.** The compound as shown in Formula I according to claim 1, **characterized in that** it satisfies any one of the following conditions:

(1) $R^2$ and $R^3$ are H, and $R^1$ is a $C_{1-6}$ alkyl substituted with one or more $R^{1-1}$; preferably, $R^2$ and $R^3$ are H, and $R^1$ is

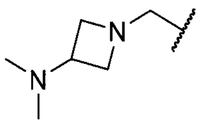 or 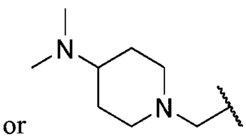 ;

(2) $R^1$ and $R^2$ are H, and $R^3$ is a $C_{1-6}$ alkyl substituted with one or more $R^{1-1}$; preferably, $R^1$ and $R^2$ are H, and $R^3$ is

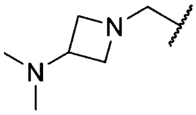 , 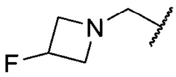 , 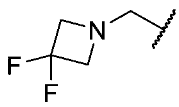 or 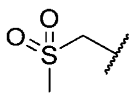 .

**4.** The compound as shown in Formula I according to claim 1, **characterized in that** the compound as shown in Formula I is any one of the following compounds:

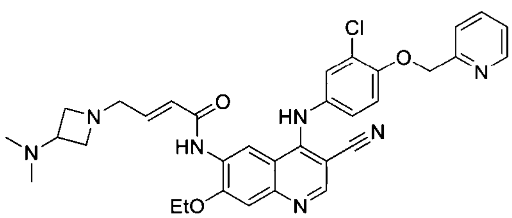 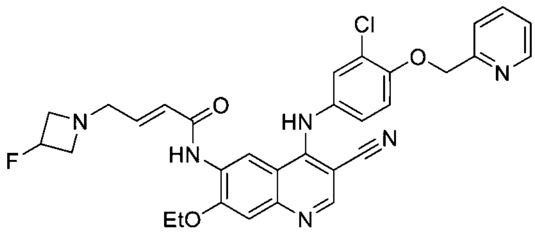

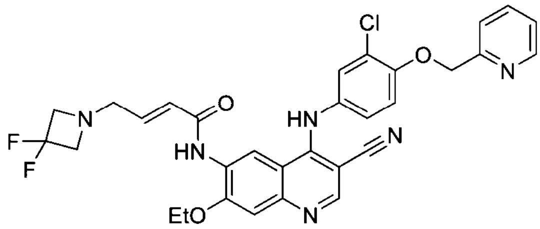 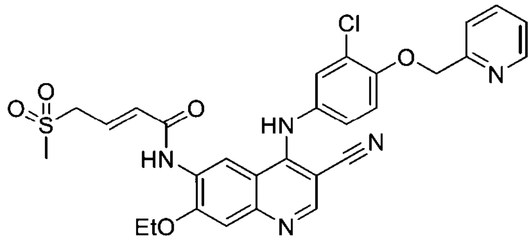

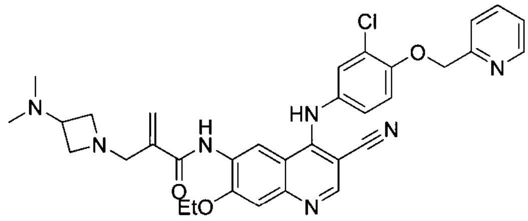 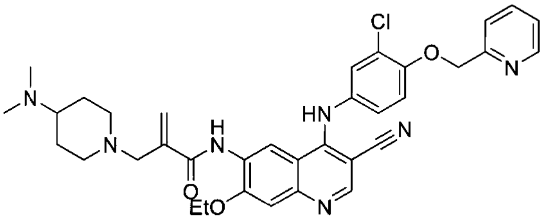 .

5. A pharmaceutical composition 1, comprising a compound as shown in Formula I according to claim 1, or a pharmaceutically acceptable salt thereof, and a pharmaceutically acceptable excipient.

6. A pharmaceutical composition 2, comprising particles, wherein the particles comprise the following components in the following mass fractions: 30-45% of a compound of formula II or a pharmaceutically acceptable salt thereof, 7-28% of copovidone, 25-48% of a filler, 1.0-8.0% of a disintegrant, 0.5-4.0% of a glidant, and 0.5-4.0% of a lubricant; wherein the sum of the mass fractions of all components in the particles is 100%; and the mass fraction refers to the percentage by mass of each component in the particles relative to the total mass of all components;

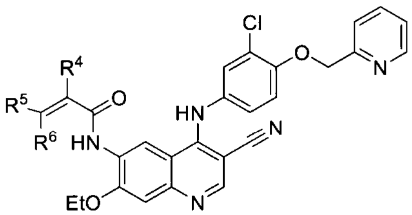

II ;

wherein:

$R^4$, $R^5$, and $R^6$ are each independently H or a $C_{1-6}$ alkyl substituted with one or more $R^{4-1}$;
each $R^{4-1}$ is independently -N($C_{1-6}$ alkyl)$_2$, a 4-6 membered heterocycloalkyl in which the heteroatoms are selected from one, two, or three of N, O, and S, with one, two, or three heteroatoms, a 4-6 membered heterocycloalkyl substituted with one or more $R^{4-a}$ in which the heteroatoms are selected from one, two, or three of N, O, and S, with one, two, or three heteroatoms, or -$SO_2$-$C_{1-6}$ alkyl;
each $R^{4-a}$ is independently halogen or -N($C_{1-6}$ alkyl)$_2$.

7. The compound as shown in Formula II according to claim 6, **characterized in that** it satisfies one or more of the following conditions:

(1) each $R^{4-1}$ is independently -N($C_{1-6}$ alkyl)$_2$, a 4-6 membered heterocycloalkyl substituted with one or more $R^{4-a}$ in which the heteroatoms are selected from one, two, or three of N, O, and S, with one, two, or three heteroatoms, or -$SO_2$-$C_{1-6}$ alkyl;
(2) among $R^4$, $R^5$, $R^6$, $R^{4-1}$, and $R^{4-a}$, the $C_{1-6}$ alkyl in "$C_{1-6}$ alkyl substituted with one or more $R^{4-1}$," "-$SO_2$-$C_{1-6}$ alkyl," and "-N($C_{1-6}$ alkyl)$_2$" are each independently methyl, ethyl, n-propyl, isopropyl, n-butyl, isobutyl, or tert-butyl, preferably methyl;
(3) among $R^{4-1}$, the 4-6 membered heterocycloalkyl in which the heteroatoms are selected from one, two, or three of N, O, and S, with one, two, or three heteroatoms, and in the 4-6 membered heterocycloalkyl substituted with one or more in which the heteroatoms are selected from one, two, or three of N, O, and S, with one, two, or three

heteroatoms, are each independently a 4-6 membered heterocycloalkyl containing one N heteroatom, preferably

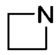 or 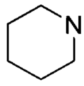 ;

(4) among $R^{4\text{-}a}$, the halogen is fluorine, chlorine, bromine, or iodine, preferably fluorine.

**8.** The compound as shown in Formula II according to claim 6, **characterized in that** it satisfies one or more of the following conditions:

(1) $R^5$ and $R^6$ are H, and $R^4$ is a $C_{1\text{-}6}$ alkyl substituted with one or more $R^{4\text{-}1}$; preferably, $R^5$ and $R^6$ are H, and $R^4$ is

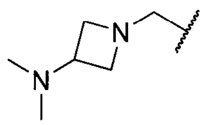 or 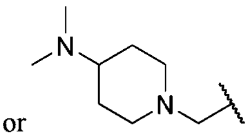 ;

(2) $R^4$ and $R^6$ are H, and $R^5$ is a $C_{1\text{-}6}$ alkyl substituted with one or more $R^{4\text{-}1}$; preferably, $R^4$ and $R^6$ are H, and $R^5$ is

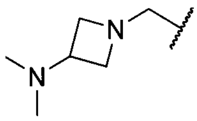 , 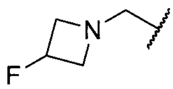 , 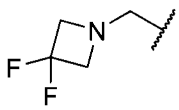 , 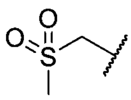 or 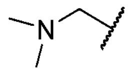 .

**9.** The pharmaceutical composition 2 according to claim 6, wherein the compound of formula II is any one of the following compounds:

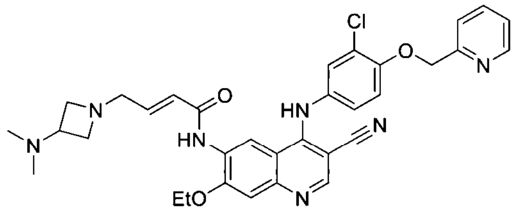

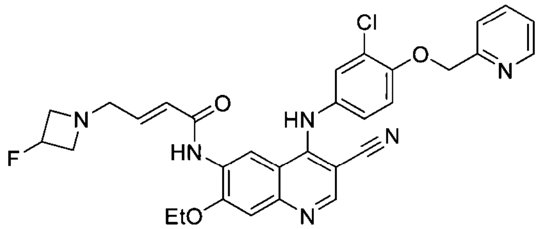

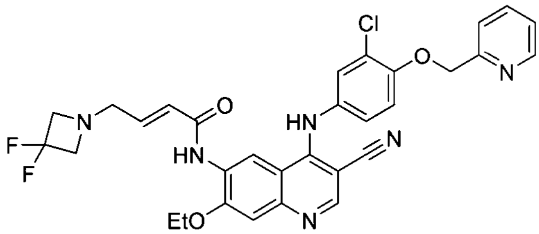

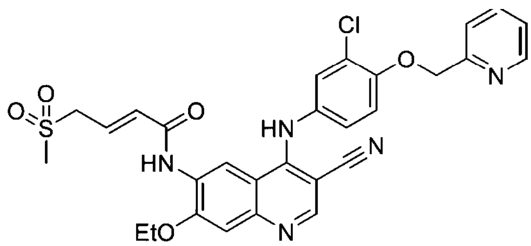

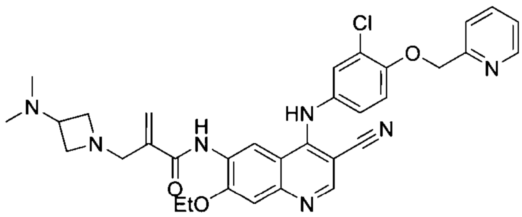

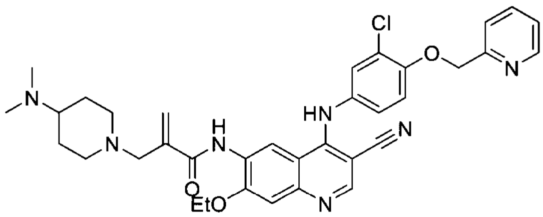

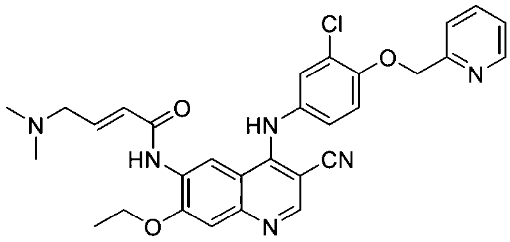

.

**10.** The pharmaceutical composition 2 according to claim 6, **characterized in that** it satisfies one or more of the following conditions:

(1) In the pharmaceutical composition, the pharmaceutically acceptable salt is the maleate salt of the compound of formula II;
(2) In the pharmaceutical composition, the mass fraction of the compound of formula II or its pharmaceutically acceptable salt is 35-42%; preferably 36%;
(3) In the pharmaceutical composition, the mass fraction of copovidone is 8-22%; preferably 21%;
(4) In the pharmaceutical composition, the filler is a sugar alcohol and/or a water-swellable additive; preferably mannitol and microcrystalline cellulose;
(5) In the pharmaceutical composition, the mass fraction of the filler is 34-45%; preferably 37%;
(6) In the pharmaceutical composition, the disintegrant is one or more selected from the group consisting of adipic acid, alginic acid, gelatinized starch, carboxymethyl starch sodium, carboxymethyl cellulose, calcium carboxymethyl cellulose, sodium carboxymethyl cellulose, hydrated silica, calcium citrate, cross-linked sodium carboxymethyl cellulose, crospovidone, light anhydrous silicic acid, crystalline cellulose, synthetic aluminum silicate, wheat starch, rice starch, hydroxypropyl methylcellulose, hydroxypropyl starch, pregelatinized starch, monosodium fumarate, povidone, anhydrous citric acid, methylcellulose, and dicalcium phosphate; preferably crospovidone, more preferably crospovidone XL-10;
(7) In the pharmaceutical composition, the mass fraction of the disintegrant is 1.7-4.9%; preferably 2.0%;
(8) In the pharmaceutical composition, the glidant is one or more selected from the group consisting of colloidal silicon dioxide, gel silicon dioxide, light anhydrous silicic acid, crystalline cellulose, synthetic aluminum silicate, titanium oxide, stearic acid, calcium stearate, magnesium stearate, tricalcium phosphate, talc, corn starch, and magnesium aluminum silicate; preferably colloidal silicon dioxide or gel silicon dioxide;
(9) The mass fraction of the glidant is 1.0-4.0%; preferably 2.0%;
(10) In the pharmaceutical composition, the lubricant is one or more selected from the group consisting of cocoa butter, Brazil wax, hydrated silica (colloidal silicon dioxide), aluminum hydroxide dry gel, glycerol fatty acid esters, magnesium silicate, light anhydrous silicic acid, crystalline cellulose, hydrogenated oil, synthetic aluminum silicate, white beeswax, magnesium oxide, sodium potassium tartrate, sucrose fatty acid esters, stearic acid, calcium stearate, magnesium stearate, sodium stearyl fumarate, stearyl alcohol, and polyethylene glycol 40 stearate; preferably sodium stearyl fumarate or magnesium stearate;
(11) The mass fraction of the lubricant is 1.0-4.0%; preferably 2%;
(12) In the pharmaceutical composition, the pharmaceutically acceptable salt exists in an anhydrous crystalline or amorphous form; preferably in an anhydrous crystalline form.

**11.** The pharmaceutical composition 2 according to claim 6, wherein it comprises particles, wherein the particles comprise the following components in the following mass fractions: 30-45% anhydrous neratinib maleate, 7-28% copovidone, 25-48% filler, 1.0-8.0% disintegrant, 0.5-4.0% glidant, and 0.5-4.0% lubricant; the total mass fraction of all components in the particles is 100% optionally wherein it satisfies one or more of the following conditions:

(1) In the pharmaceutical composition 2, the anhydrous neratinib maleate in the composition is a crystalline form, having characteristic peaks in its X-ray powder diffraction pattern at 2θ of 6.0±0.2°, 7.3±0.2°, 10.1±0.2°, 12.1 ±0.2°, 15.6±0.2°, 17.3±0.2° and 19.9±0.2°; preferably, the XRPD pattern of the crystalline form of neratinib maleate is substantially as shown in Figures 1, 2 or 3;
(2) In the pharmaceutical composition 2, the mass fraction of the anhydrous neratinib maleate is 35-42%; preferably 36%;
(3) In the pharmaceutical composition 2, the mass fraction of copovidone is 8-22%; preferably 21%;
(4) In the pharmaceutical composition 2, the filler is sugar alcohol and/or water-swellable additive; preferably mannitol and microcrystalline cellulose;
(5) In the pharmaceutical composition 2, the mass fraction of the filler is 34-45%; preferably 37%;

(6) In the pharmaceutical composition 2, the disintegrant is one or more selected from adipic acid, alginic acid, pregelatinized starch, sodium carboxymethyl starch, carboxymethyl cellulose, calcium carboxymethyl cellulose, sodium carboxymethyl cellulose, hydrated silicon dioxide, calcium citrate, crosslinked sodium carboxymethyl cellulose, crospovidone, light anhydrous silica, crystalline cellulose, synthetic aluminum silicate, wheat starch, rice starch, cellulose acetate phthalate, calcium stearate, low-substituted hydroxypropyl cellulose, corn starch, tragacanth gum, potato starch, hydroxyethyl methylcellulose, hydroxypropyl starch, pregelatinized starch, monosodium fumarate, povidone, anhydrous citric acid, methylcellulose and calcium dihydrogen phosphate; preferably crospovidone, more preferably crospovidone XL-10;
(7) In the pharmaceutical composition 2, the mass fraction of the disintegrant is 1.7-4.9%; preferably 2.0%;
(8) In the pharmaceutical composition 2, the glidant is one or more selected from colloidal silicon dioxide, gel silicon dioxide, light anhydrous silica, crystalline cellulose, synthetic aluminum silicate, titanium oxide, stearic acid, calcium stearate, magnesium stearate, tricalcium phosphate, talc, corn starch and magnesium aluminum silicate; preferably colloidal silicon dioxide or gel silicon dioxide;
(9) In the pharmaceutical composition 2, the mass fraction of the glidant is 1.0-4.0%; preferably 2.0%;
(10) In the pharmaceutical composition 2, the lubricant is one or more selected from cocoa butter, Brazilian palm wax, hydrated silicon dioxide (colloidal silicon dioxide), aluminum hydroxide dry gel, glycerol fatty acid esters, magnesium silicate, light anhydrous silica, crystalline cellulose, hydrogenated oil, synthetic aluminum silicate, beeswax, magnesium oxide, potassium sodium tartrate, sucrose fatty acid esters, stearic acid, calcium stearate, magnesium stearate, sodium stearyl fumarate, stearyl alcohol and polyethylene glycol 40 stearate; preferably sodium stearyl fumarate or magnesium stearate;
(11) In the pharmaceutical composition 2, the mass fraction of the lubricant is 1.0-4.0%; preferably 2%;
(12) In the pharmaceutical composition 2, the composition further comprises a coating agent; the coating agent is preferably one or more selected from hydroxypropyl methylcellulose, methylcellulose, ethylcellulose, hydroxypropyl cellulose, polyvinyl alcohol, povidone, polyvinyl acetate resin, polyvinyl alcohol-ethylene amine acetal, methacrylic acid-alkyl amino copolymer RS, ethyl acrylate-methyl methacrylate copolymer dispersion, sucrose, mannitol and Opadry; more preferably Opadry; the mass ratio of the coating agent to the pharmaceutical particles is preferably 0.01:1-0.05:1, e.g., 0.03:1, 0.034:1 or 0.036:1;

Optionally the pharmaceutical composition is a solid formulation.

**12.** The pharmaceutical composition 2 according to claim 11, wherein it satisfies one or more of the following conditions: the composition comprises granules, wherein the components of the granules comprise an inner components and outer components;

the inner components of the granules: 30-45% anhydrous neratinib maleate, 7-28% copovidone, 25-48% filler, 1.0-8.0% disintegrant, 1.0-4.0% glidant, and 0.5-4.0% lubricant;
the outer components of the granules: 0-2.5% disintegrant and/or 0-1% lubricant, wherein the disintegrant and the lubricant are not simultaneously 0;
preferably, the granules comprise components from group (1), group (2), or group (3):

Group (1):

inner components of the granules: 34.9% anhydrous neratinib maleate, 26.2% mannitol, 8.3% microcrystalline cellulose, 21.7% copovidone, 2.5% crospovidone XL-10, 2.0% colloidal silicon dioxide, and 1.0% magnesium stearate;
outer components of the granules: 2.5% crospovidone XL-10 and 1.0% magnesium stearate;

Group (2):

inner components of the granules: 42.3% anhydrous neratinib maleate, 31.7% mannitol, 13.1% microcrystalline cellulose, 8.0% copovidone, 2.0% crospovidone XL-10, 2.0% colloidal silicon dioxide, and 1.0% magnesium stearate;
outer components of the granules: 1.0% magnesium stearate;

Group (3):

inner components of the granules: 36.1% anhydrous neratinib maleate, 27.1% mannitol, 8.6% microcrystalline cellulose, 22.4% copovidone, 1.7% crospovidone XL-10, 2.0% colloidal silicon dioxide, and

1.0% magnesium stearate;
outer components of the granules: 1.0% magnesium stearate.

**13.** The pharmaceutical composition 2 according to claim 12, wherein it comprises components of one of the following group (a), group (b), or group (c):

Group (a): granules and Opadry, wherein the mass ratio of Opadry to the granules is 0.03:1;

inner components of the granules: 34.9% anhydrous neratinib maleate, 26.2% mannitol, 8.3% microcrystalline cellulose, 21.7% copovidone, 2.5% crospovidone XL-10, 2.0% colloidal silicon dioxide, and 1.0% magnesium stearate;
outer components of the granules: 2.5% crospovidone XL-10 and 1.0% magnesium stearate;

Group (b): granules and Opadry, wherein the mass ratio of Opadry to the granules is 0.034:1;

inner components of the granules: 42.3% anhydrous neratinib maleate, 31.7% mannitol, 13.1% microcrystalline cellulose, 8.0% copovidone, 2.0% crospovidone XL-10, 2.0% colloidal silicon dioxide, and 1.0% magnesium stearate;
outer components of the granules: 1.0% magnesium stearate;

Group (c): granules and Opadry, wherein the mass ratio of Opadry to the granules is 0.036:1;

inner components of the granules: 36.1% anhydrous neratinib maleate, 27.1% mannitol, 8.6% microcrystalline cellulose, 22.4% copovidone, 1.7% crospovidone XL-10, 2.0% colloidal silicon dioxide, and 1.0% magnesium stearate;
outer components of the granules: 1.0% magnesium stearate.

**14.** A method for preparing the pharmaceutical composition 2 according to any one of claims 11-13, wherein it comprises the following step: granulating the following raw materials in the indicated mass fractions to obtain granules;

the raw materials comprise 30-45% anhydrous neratinib maleate, 7-28% copovidone, 25-48% filler, 1.0-8.0% disintegrant, 0.5-4.0% glidant, and 0.5-4.0% lubricant; the sum of the mass fractions of all components in the granules is 100%; the mass fractions refers to the mass of each component in the granules as a percentage of the total mass of all components optionally_wherein it satisfies one or more of the following conditions:

(1) the neratinib maleate is anhydrous neratinib maleate, and the type and content of the anhydrous neratinib maleate are as described in any one of claims 11-14;
(2) the copovidone,, the filler, the disintegrant, the glidant, and the lubricant are as described in any one of claims 11-14;
(3) the granulation method is dry granulation, and the dry granulation is carried out using a dry granulator;

optionally the method further comprises a Step 2:

Step 2: tableting the granules obtained as described above with the external raw materials of the granules to obtain the granules;
the external raw materials of the granules comprise 0-2.5% disintegrant and/or 0-1% lubricant, wherein the disintegrant and the lubricant are not simultaneously 0.

**15.** Substance A for use in preventing or treating EGFR-related diseases, wherein the substance A is the compound of formula I or a pharmaceutically acceptable salt thereof according to claim 1, the pharmaceutical composition 2 according to any one of claims 11-14, or the solid pharmaceutical formulation according to claim 20.

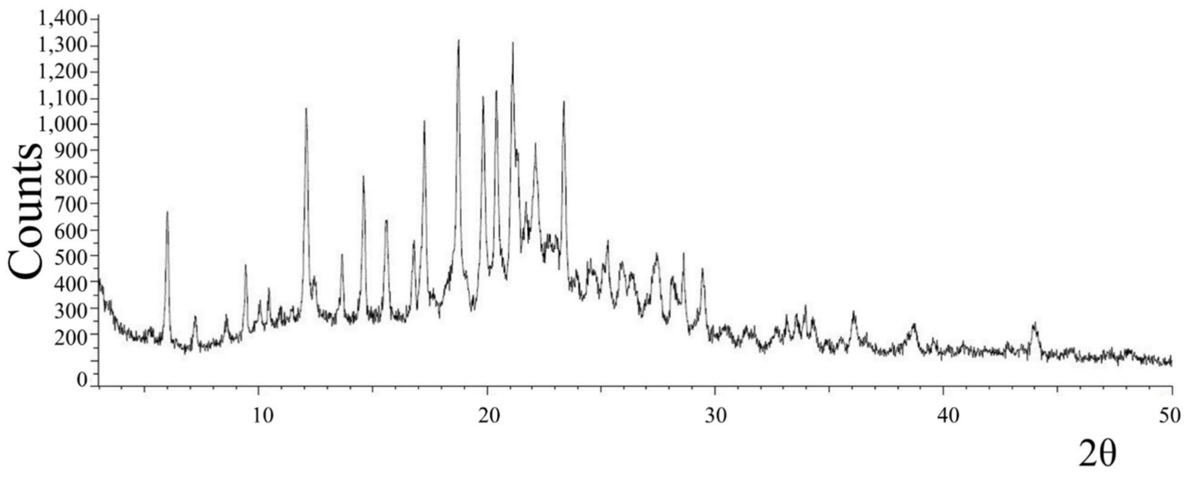
Counts
1,400
1,300
1,200
1,100
1,000
900
800
700
600
500
400
300
200
0
10
20
30
40
50
2θ

Fig. 1

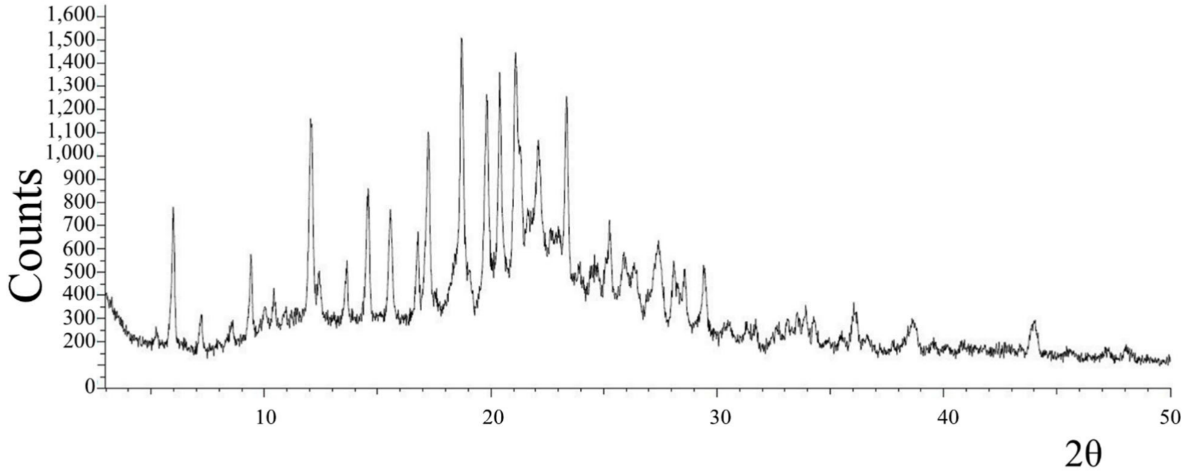
Counts
1,600
1,500
1,400
1,300
1,200
1,100
1,000
900
800
700
600
500
400
300
200
0
10
20
30
40
50
2θ

Fig. 2

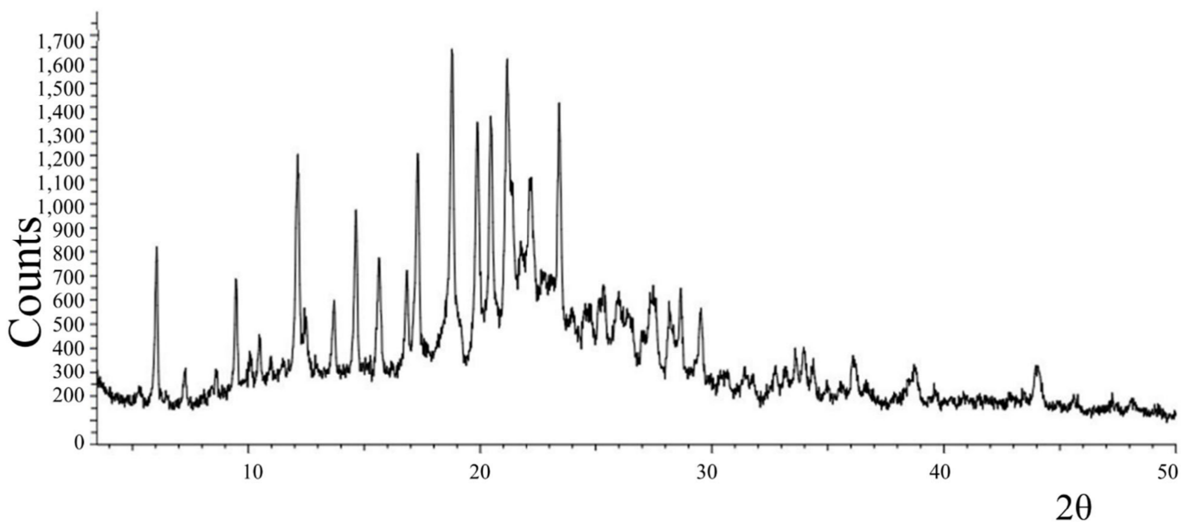
Counts
1,700
1,600
1,500
1,400
1,300
1,200
1,100
1,000
900
800
700
600
500
400
300
200
0
10
20
30
40
50
2θ

1

Fig. 3

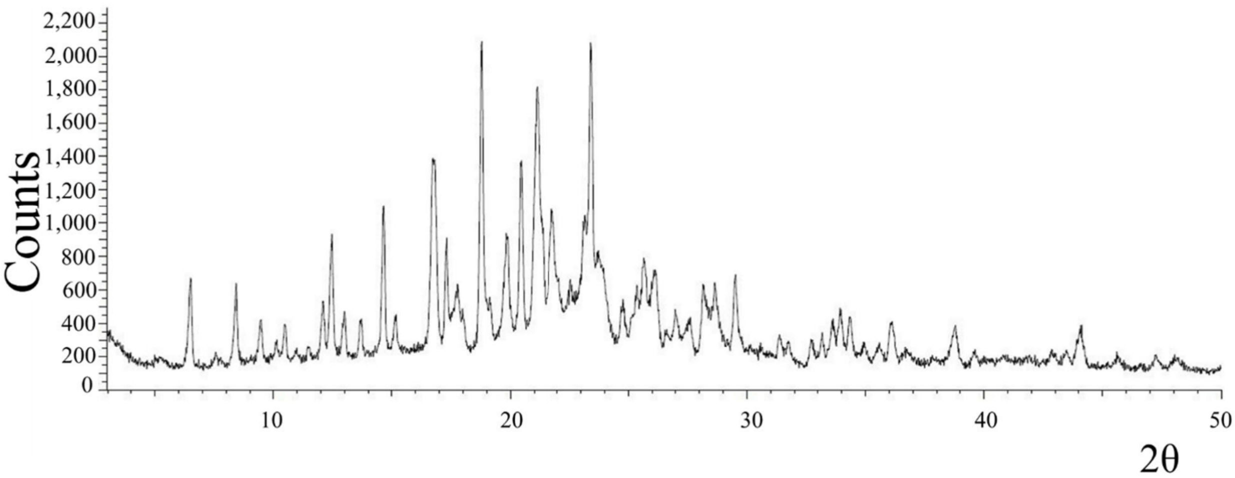


Fig. 4

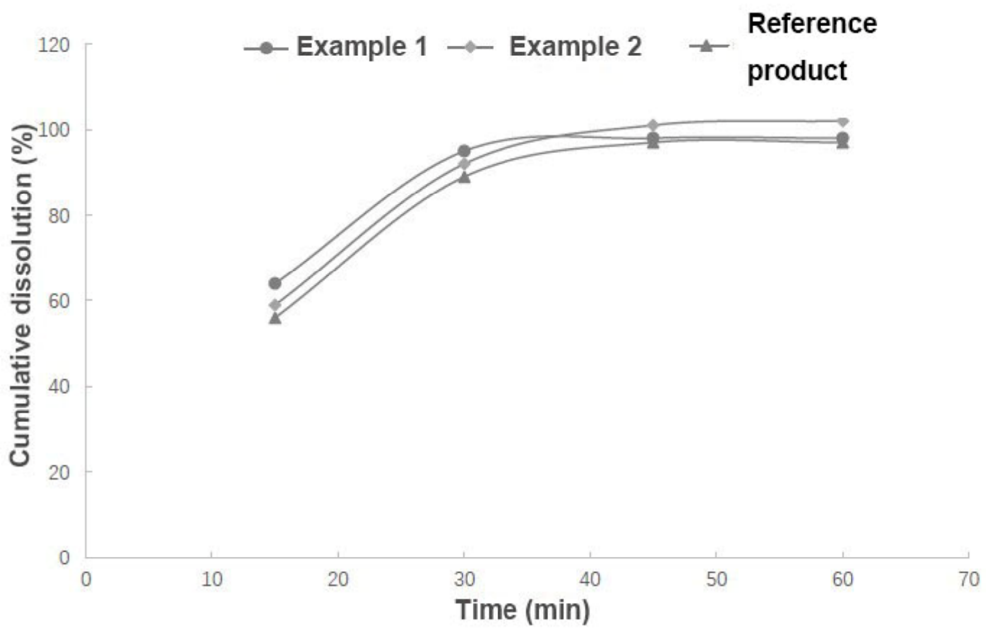


Fig. 5

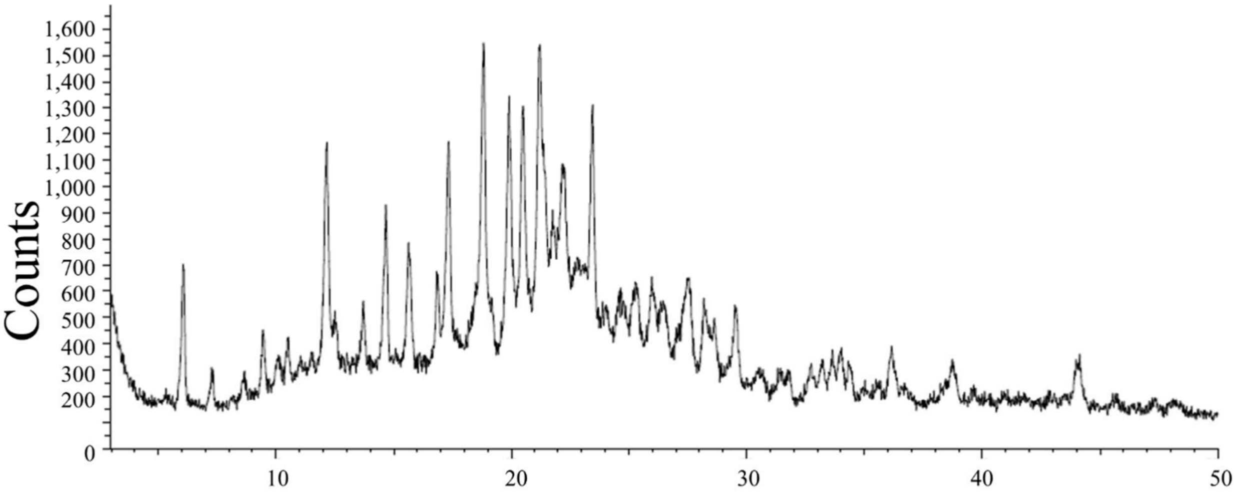


Fig. 6

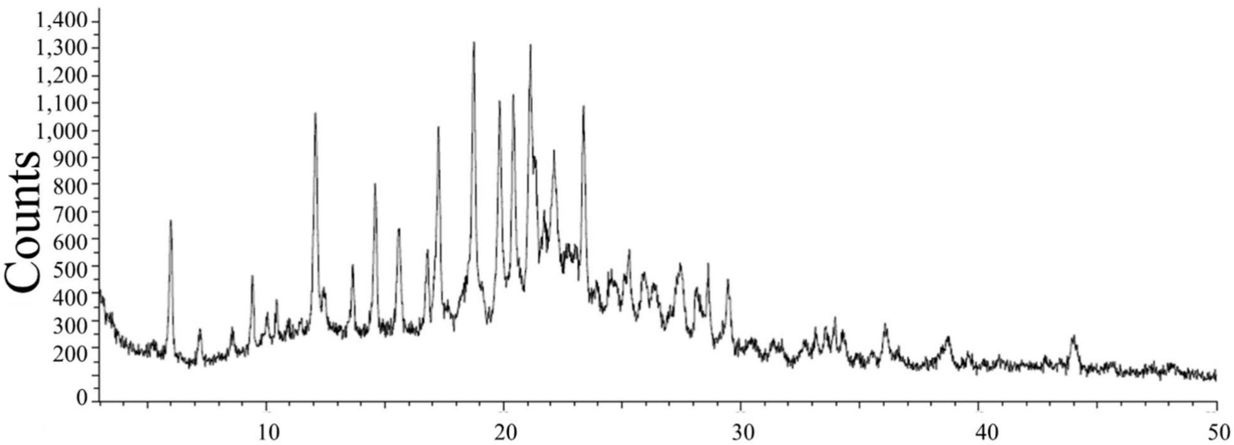


Fig. 7

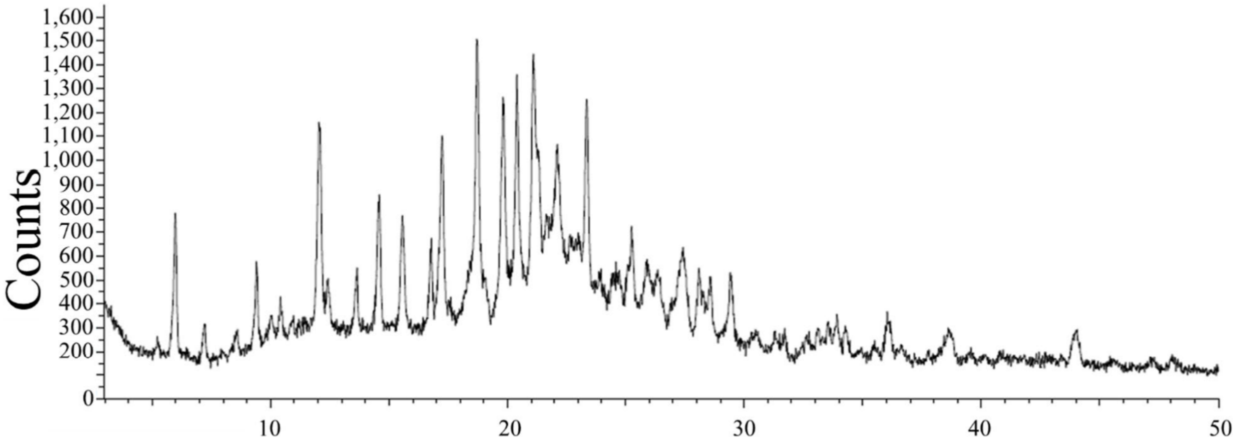


Fig. 8

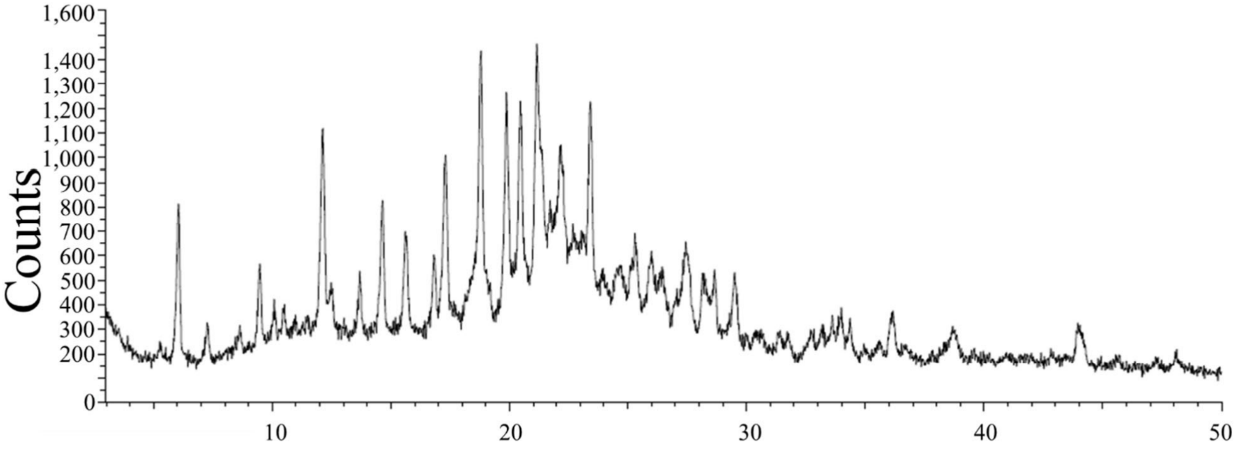


Fig. 9

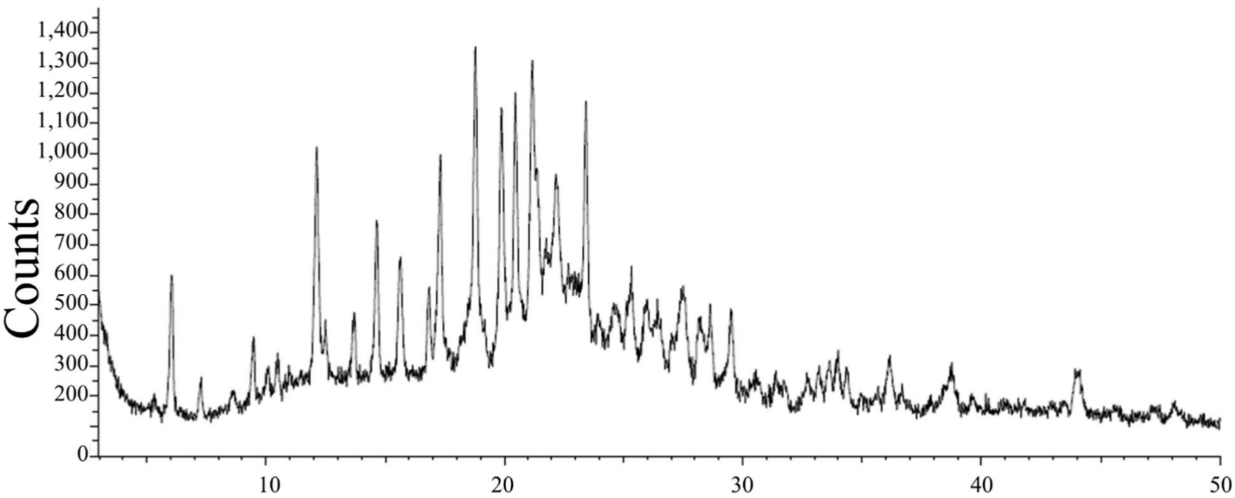


Fig. 10

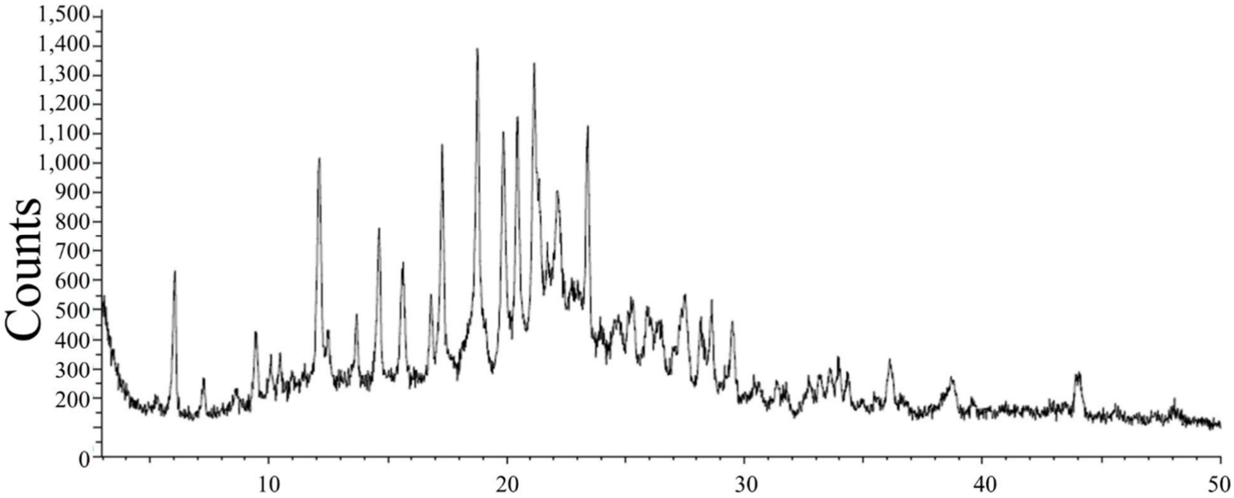


Fig. 11

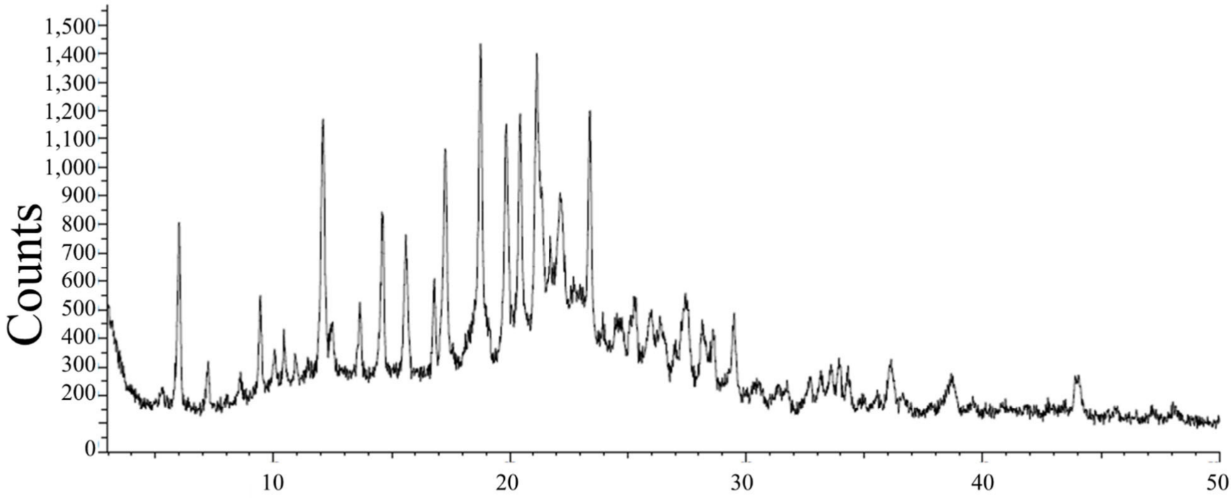


Fig. 12

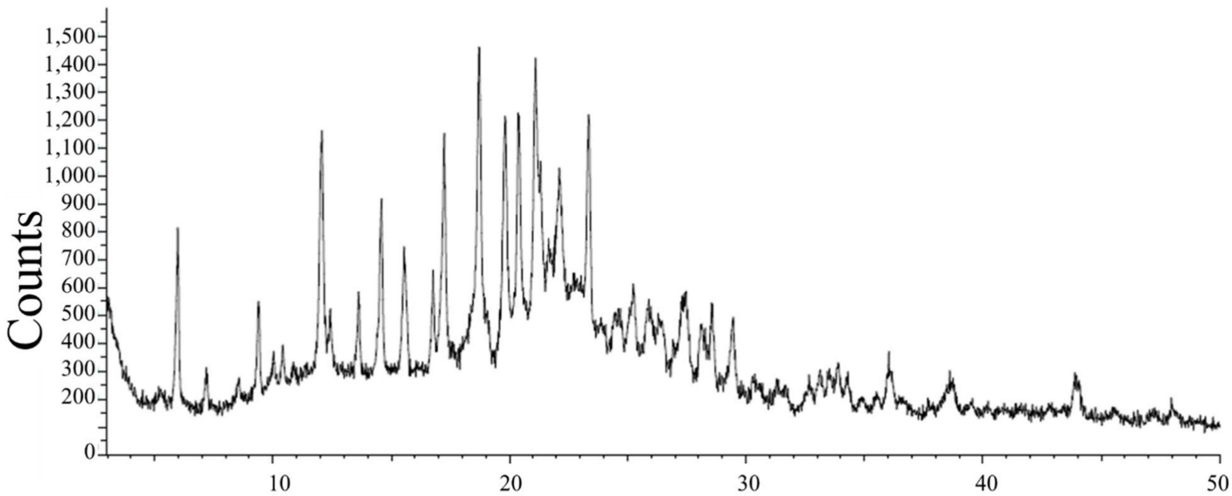


Fig. 13

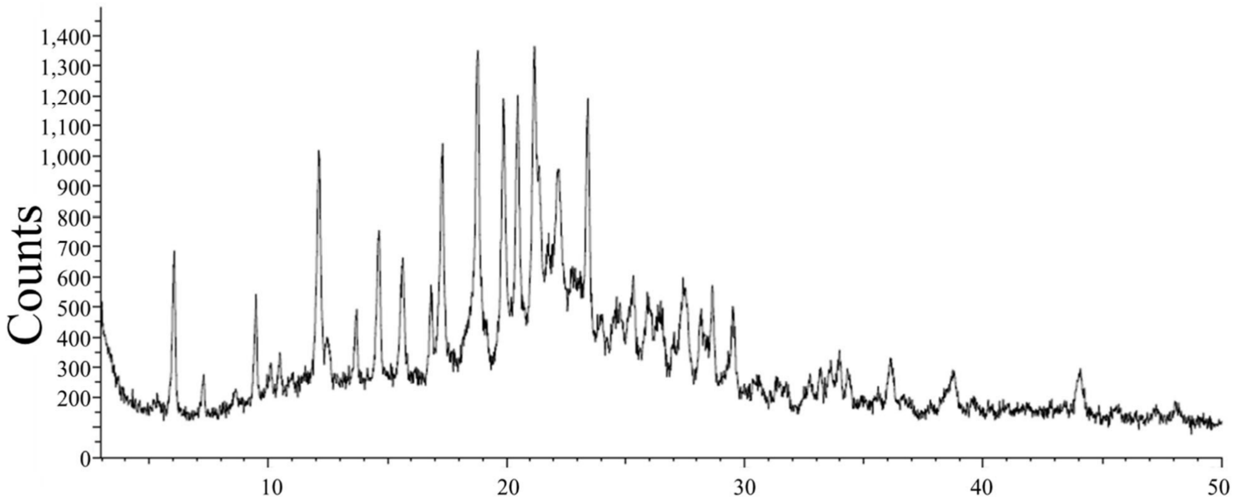


Fig. 14

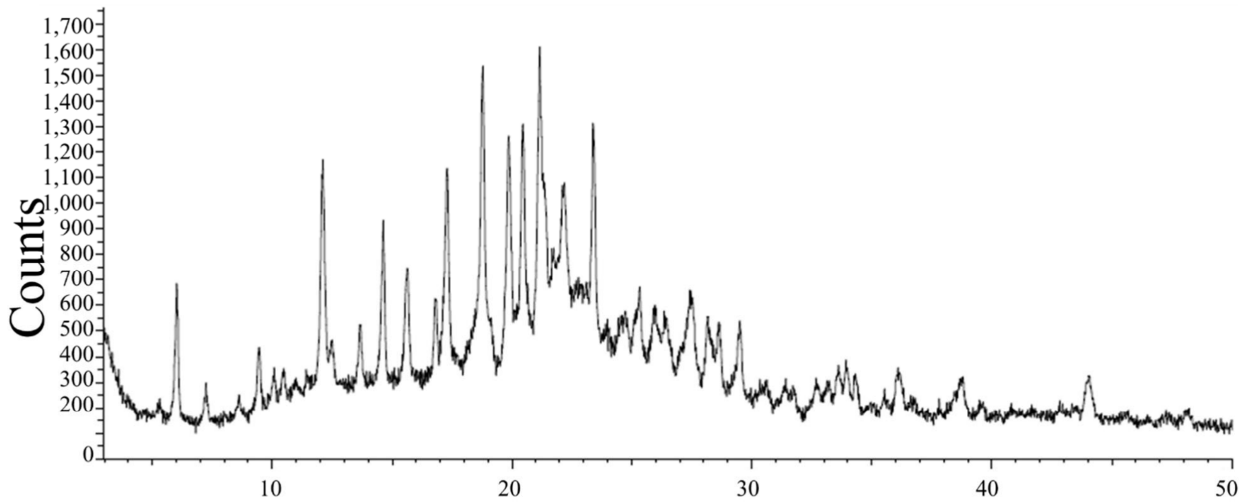
Counts
1,700
1,600
1,500
1,400
1,300
1,200
1,100
1,000
900
800
700
600
500
400
300
200
0
10
20
30
40
50

Fig. 15

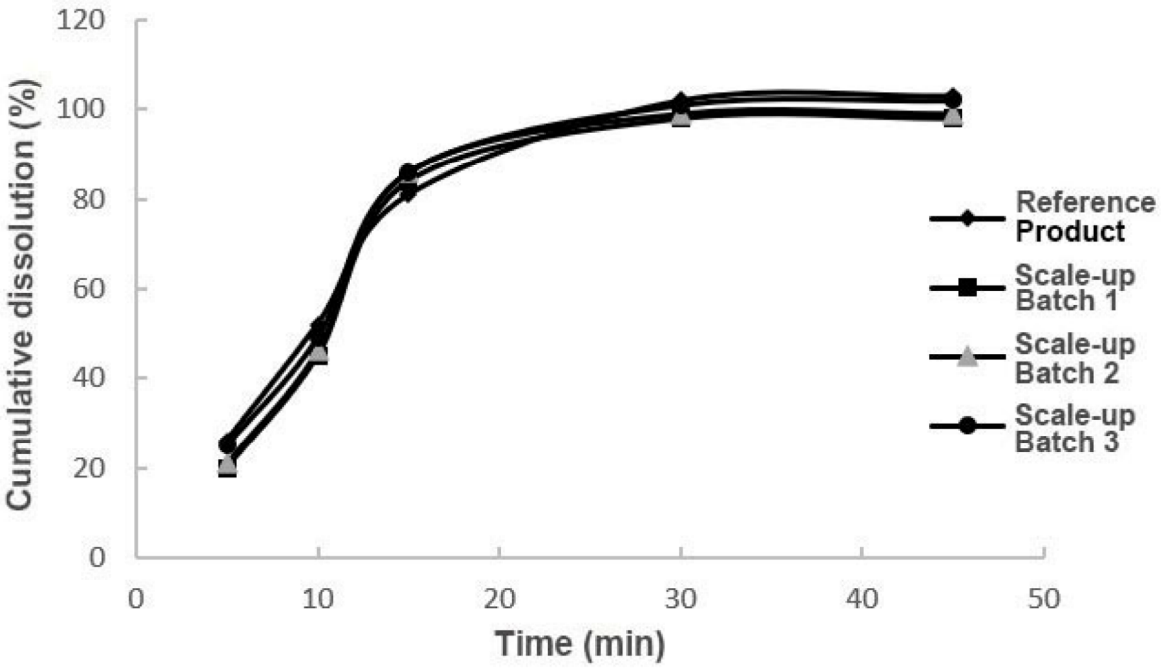
Cumulative dissolution (%)
120
100
80
60
40
20
0
0
10
20
30
40
50
Time (min)
Reference Product
Scale-up Batch 1
Scale-up Batch 2
Scale-up Batch 3

Fig. 16

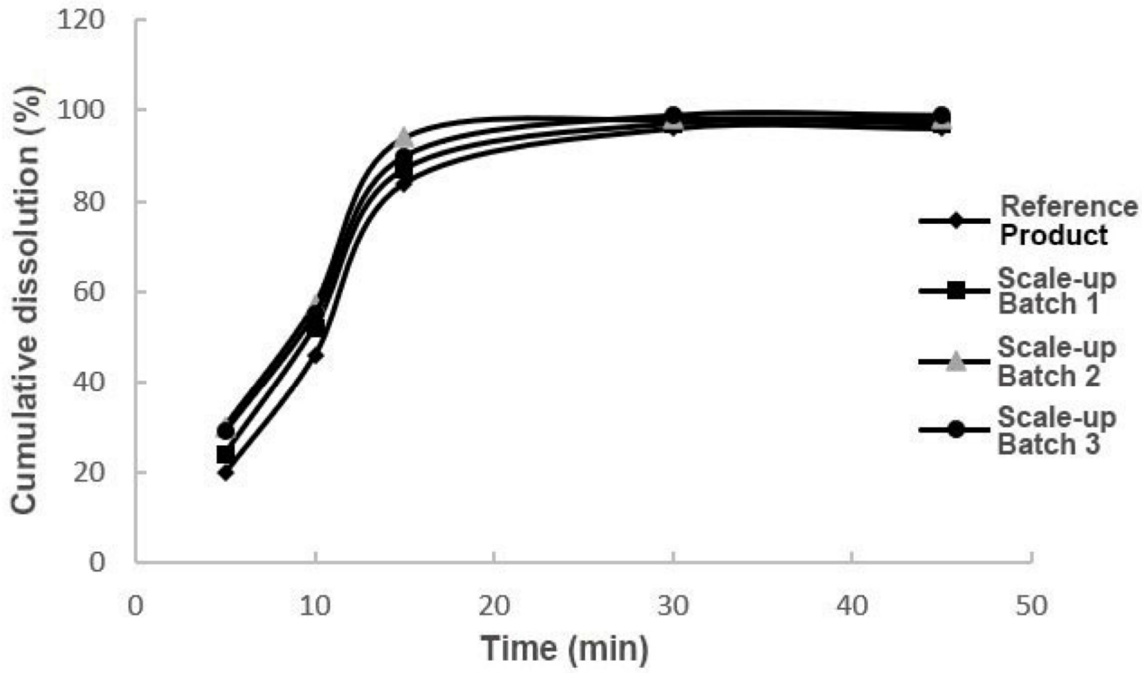

120
100
80
60
40
20
0
Cumulative dissolution (%)
0
10
20
30
40
50
Time (min)
Reference Product
Scale-up Batch 1
Scale-up Batch 2
Scale-up Batch 3


Fig. 17

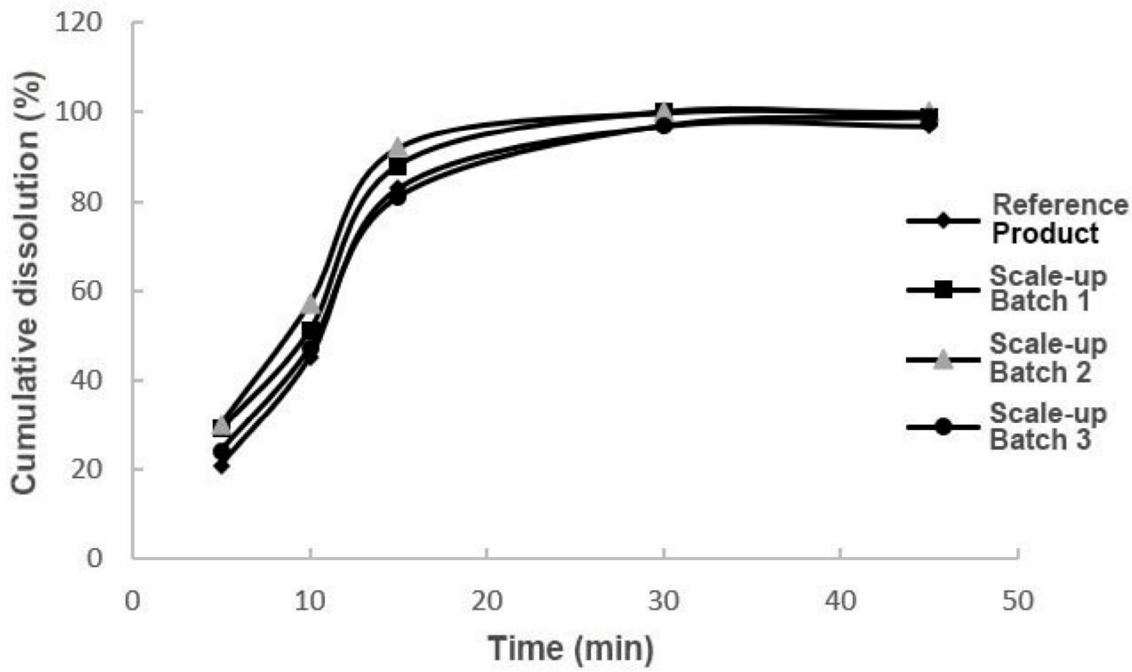

120
100
80
60
40
20
0
Cumulative dissolution (%)
0
10
20
30
40
50
Time (min)
Reference Product
Scale-up Batch 1
Scale-up Batch 2
Scale-up Batch 3


Fig. 18

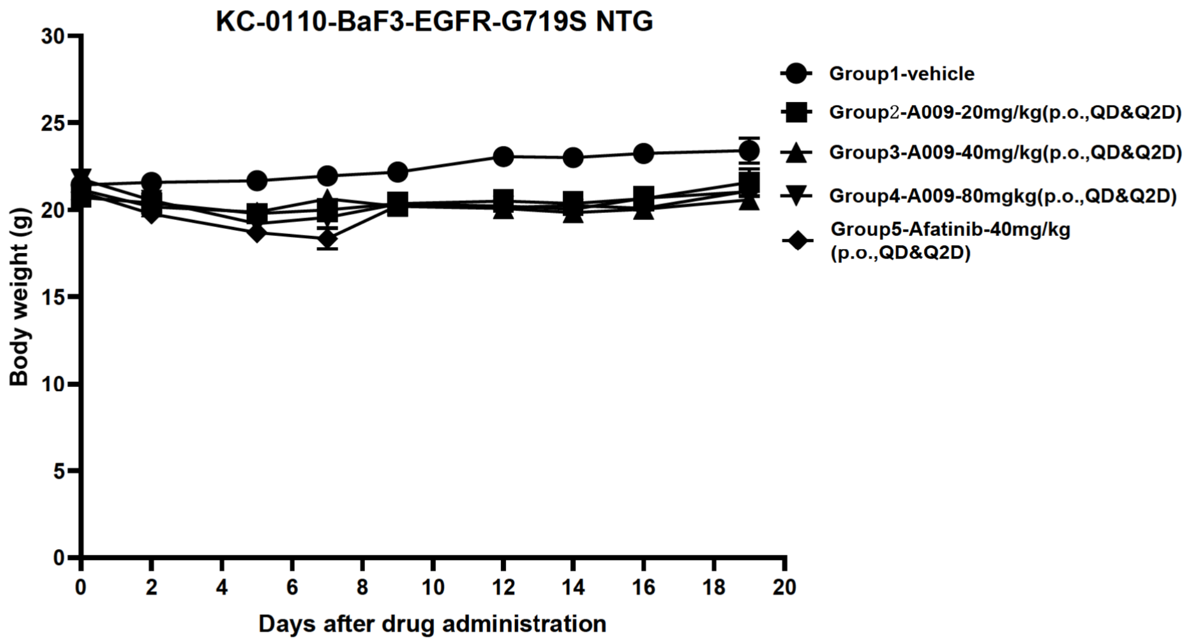
KC-0110-BaF3-EGFR-G719S NTG
Body weight (g)
Days after drug administration
Group1-vehicle
Group2-A009-20mg/kg(p.o.,QD&Q2D)
Group3-A009-40mg/kg(p.o.,QD&Q2D)
Group4-A009-80mgkg(p.o.,QD&Q2D)
Group5-Afatinib-40mg/kg
(p.o.,QD&Q2D)

Fig. 19

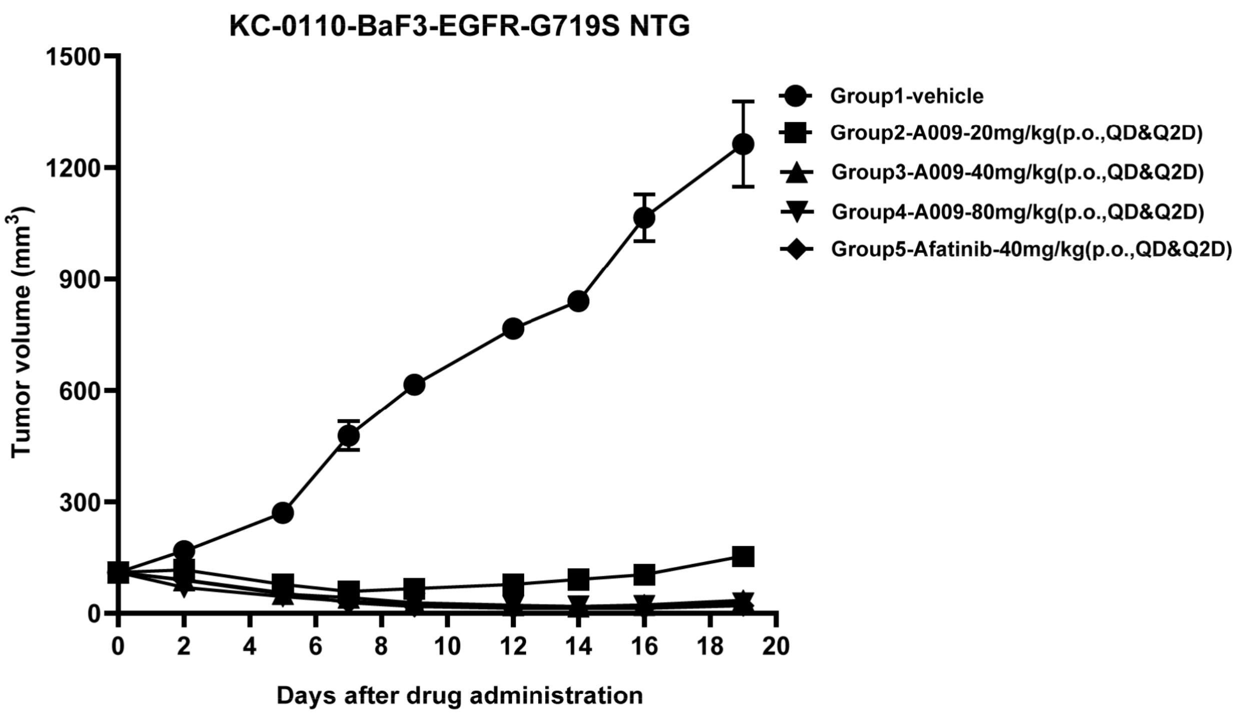
KC-0110-BaF3-EGFR-G719S NTG
Tumor volume (mm3)
Days after drug administration
Group1-vehicle
Group2-A009-20mg/kg(p.o.,QD&Q2D)
Group3-A009-40mg/kg(p.o.,QD&Q2D)
Group4-A009-80mg/kg(p.o.,QD&Q2D)
Group5-Afatinib-40mg/kg(p.o.,QD&Q2D)

Fig. 20

**INTERNATIONAL SEARCH REPORT**

International application No.
**PCT/CN2023/080921**

**A. CLASSIFICATION OF SUBJECT MATTER**

C07D 401/14(2006.01)i; C07D 401/12(2006.01)i; A61K 9/16(2006.01)i; A61K 31/4709(2006.01)i; A61K 31/343(2006.01)i; A61K 31/5377(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D A61K A61P

Documentation searched other than minimum documentation to the extent that such documents are included in the fields searched

Electronic data base consulted during the international search (name of data base and, where practicable, search terms used)

CNTXT, CNKI, ENTXT, STN(REGISTRY, MARPAT, CAPLUS): 甫康, 奈拉替尼, 来那替尼, 共聚维酮, 组合物, 干法, 肿瘤, 结构检索, EGFR, neratinib, copovidone, composition, dry, granul+, tumor, cancer

**C. DOCUMENTS CONSIDERED TO BE RELEVANT**

| Category* | Citation of document, with indication, where appropriate, of the relevant passages | Relevant to claim No. |
|---|---|---|
| E | CN 115806548 A (FUKANG (SHANGHAI) HEALTH TECHNOLOGY CO., LTD.) 17 March 2023 (2023-03-17)<br>claims 1-24, and embodiments | 1-24 |
| X | CARMI, Caterina et al. "Irreversible Inhibition of Epidermal Growth Factor Receptor Activity by 3-Aminopropanamides"<br>*Journal of Medicinal Chemistry,* Vol. 55, 26 January 2012 (2012-01-26), 2251-2264<br>abstract, and table 1 | 1-2, 5 |
| X | CN 103965120 A (SHANGHAI PHARMACEUTICALS HOLDING CO., LTD. et al.) 06 August 2014 (2014-08-06)<br>claims 1-17 and 21-22 | 1-5, 21 |
| Y | CN 103965120 A (SHANGHAI PHARMACEUTICALS HOLDING CO., LTD. et al.) 06 August 2014 (2014-08-06)<br>claims 1-17 and 21-22 | 6-21, |
| X | CN 101880273 A (SHANGHAI HENGRUI PHARMACEUTICAL CO., LTD.) 10 November 2010 (2010-11-10)<br>claims 1-11 | 1-5, 21 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 November 2023** | **24 November 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | <br>Telephone No. |

Form PCT/ISA/210 (second sheet) (July 2022)

**INTERNATIONAL SEARCH REPORT**

International application No.
**PCT/CN2023/080921**

**C. DOCUMENTS CONSIDERED TO BE RELEVANT**

| Category* | Citation of document, with indication, where appropriate, of the relevant passages | Relevant to claim No. |
|---|---|---|
| Y | CN 101880273 A (SHANGHAI HENGRUI PHARMACEUTICAL CO., LTD.) 10 November 2010 (2010-11-10) claims 1-11 | 6-21, |
| X | WO 2017186140 A1 (JIANGSU HENGRUI MEDICINE CO., LTD.) 02 November 2017 (2017-11-02) claims 1 and 8-10 | 1-5, 21 |
| Y | WO 2017186140 A1 (JIANGSU HENGRUI MEDICINE CO., LTD.) 02 November 2017 (2017-11-02) claims 1 and 8-10 | 6-21, |
| X | WO 2013131424 A1 (SHANGHAI HENGRUI PHARMACEUTICAL CO., LTD. et al.) 12 September 2013 (2013-09-12) claims 1-28 | 1-5, 21 |
| Y | WO 2013131424 A1 (SHANGHAI HENGRUI PHARMACEUTICAL CO., LTD. et al.) 12 September 2013 (2013-09-12) claims 1-28 | 6-21, |
| X | WO 2011029265 A1 (JIANGSU HENGRUI MEDICINE CO., LTD. et al.) 17 March 2011 (2011-03-17) claims 1-22 | 1-5, 21 |
| Y | WO 2011029265 A1 (JIANGSU HENGRUI MEDICINE CO., LTD. et al.) 17 March 2011 (2011-03-17) claims 1-22 | 6-21, |
| X | CN 1659145 A (WYETH CORP.) 24 August 2005 (2005-08-24) claims 1-31 | 1-5, 21 |
| Y | CN 1659145 A (WYETH CORP.) 24 August 2005 (2005-08-24) claims 1-31 | 6-21, |
| X | CN 102838539 A (SUZHOU METABOMICS, INC.) 26 December 2012 (2012-12-26) claims 1-10 | 1-5, 21 |
| Y | CN 102838539 A (SUZHOU METABOMICS, INC.) 26 December 2012 (2012-12-26) claims 1-10 | 6-21, |
| X | CN 102724970 A (WYETH LLC) 10 October 2012 (2012-10-10) embodiments 1-4, and description, paragraphs 21-26 and 34-45 | 6-21, |
| Y | CN 102724970 A (WYETH LLC) 10 October 2012 (2012-10-10) embodiments 1-4, and description, paragraphs 21-26 and 34-45 | 6-24 |
| Y | CN 103242273 A (SHANGHAI INSTITUTE OF MATERIA MEDICA, CHINESE ACADEMY OF SCIENCES) 14 August 2013 (2013-08-14) claims 1-5 and 7-8 | 22, 24 |
| Y | CN 102209714 A (UNIVERSITY OF BASEL) 05 October 2011 (2011-10-05) claims 8, 14, and 21-22 | 23-24 |

Form PCT/ISA/210 (second sheet) (July 2022)

**INTERNATIONAL SEARCH REPORT**
**Information on patent family members**

International application No.
**PCT/CN2023/080921**

| Patent document cited in search report | Publication date (day/month/year) | Patent family member(s) | Publication date (day/month/year) |
|---|---|---|---|
| CN 115806548 A | 17 March 2023 | None | |
| CN 103965120 A | 06 August 2014 | None | |
| CN 101880273 A | 10 November 2010 | None | |
| WO 2017186140 A1 | 02 November 2017 | DK 3447051 T3 | 17 January 2022 |
| | | US 2020392111 A1 | 17 December 2020 |
| | | US 11198683 B2 | 14 December 2021 |
| | | PL 3447051 T3 | 14 March 2022 |
| | | BR 112018071617 A2 | 19 February 2019 |
| | | US 2019127350 A1 | 02 May 2019 |
| | | US 10793548 B2 | 06 October 2020 |
| | | PT 3447051 T | 06 January 2022 |
| | | TW 201738236 A | 01 November 2017 |
| | | TWI 739825 B | 21 September 2021 |
| | | JP 2019520305 A | 18 July 2019 |
| | | JP 6947749 B2 | 13 October 2021 |
| | | HUE 057402 T2 | 28 May 2022 |
| | | ES 2903416 T3 | 01 April 2022 |
| | | CA 3021471 A1 | 02 November 2017 |
| | | EP 3447051 A1 | 27 February 2019 |
| | | EP 3447051 A4 | 27 November 2019 |
| | | EP 3447051 B1 | 15 December 2021 |
| WO 2013131424 A1 | 12 September 2013 | TW 201336835 A | 16 September 2013 |
| | | TWI 586659 B | 11 June 2017 |
| WO 2011029265 A1 | 17 March 2011 | AU 2010292790 A1 | 10 May 2012 |
| | | AU 2010292790 B2 | 26 March 2015 |
| | | EP 2479174 A1 | 25 July 2012 |
| | | EP 2479174 A4 | 17 April 2013 |
| | | EP 2479174 B1 | 18 October 2017 |
| | | ES 2647829 T3 | 26 December 2017 |
| | | CA 2774099 A1 | 17 March 2011 |
| | | CA 2774099 C | 04 July 2017 |
| | | PL 2479174 T3 | 28 February 2018 |
| | | DK 2479174 T3 | 27 November 2017 |
| | | HUE 035873 T2 | 28 May 2018 |
| | | US 2012165352 A1 | 28 June 2012 |
| | | US 8901140 B2 | 02 December 2014 |
| | | JP 2013504521 A | 07 February 2013 |
| | | JP 5684811 B2 | 18 March 2015 |
| | | LT 2479174 T | 27 November 2017 |
| | | MX 2012002875 A | 20 April 2012 |
| | | PT 2479174 T | 20 November 2017 |
| | | NO 2479174 T3 | 17 March 2018 |
| | | KR 20120081142 A | 18 July 2012 |
| | | KR 101738191 B1 | 19 May 2017 |
| | | HRP 20171748 T1 | 29 December 2017 |
| | | SI 2479174 T1 | 29 December 2017 |
| | | US 2015126550 A1 | 07 May 2015 |
| | | US 9358227 B2 | 07 June 2016 |
| | | BR 112012005760 A2 | 16 February 2016 |

Form PCT/ISA/210 (patent family annex) (July 2022)

**INTERNATIONAL SEARCH REPORT**
**Information on patent family members**

International application No.
**PCT/CN2023/080921**

| Patent document cited in search report | | | Publication date (day/month/year) | Patent family member(s) | | | Publication date (day/month/year) |
|---|---|---|---|---|---|---|---|
| | | | | RU | 2012110882 | A | 10 November 2013 |
| | | | | RU | 2536102 | C2 | 20 December 2014 |
| CN | 1659145 | A | 24 August 2005 | KR | 20050044599 | A | 12 May 2005 |
| | | | | ZA | 200405025 | B | 27 December 2006 |
| | | | | HU | 0402228 | A2 | 28 February 2005 |
| | | | | ATE | 370123 | T1 | 15 September 2007 |
| | | | | IL | 161921 | A0 | 20 November 2005 |
| | | | | JP | 2005514384 | A | 19 May 2005 |
| | | | | US | 2003149056 | A1 | 07 August 2003 |
| | | | | US | 6821988 | B2 | 23 November 2004 |
| | | | | BR | 0214485 | A | 14 September 2004 |
| | | | | TW | 200300348 | A | 01 June 2003 |
| | | | | UA | 77469 | C2 | 15 December 2006 |
| | | | | MXPA | 04004969 | A | 11 August 2004 |
| | | | | RU | 2004119414 | A | 27 February 2006 |
| | | | | RU | 2309150 | C2 | 27 October 2007 |
| | | | | DE | 60221886 | D1 | 27 September 2007 |
| | | | | ECSP | 045122 | A | 23 July 2004 |
| | | | | AR | 037438 | A1 | 10 November 2004 |
| | | | | WO | 03050090 | A1 | 19 June 2003 |
| | | | | CA | 2467573 | A1 | 19 June 2003 |
| | | | | EP | 1448531 | A1 | 25 August 2004 |
| | | | | EP | 1448531 | B1 | 15 August 2007 |
| | | | | PL | 370137 | A1 | 16 May 2005 |
| | | | | CO | 5580828 | A2 | 30 November 2005 |
| | | | | NZ | 533118 | A | 28 July 2006 |
| | | | | AU | 2002359489 | A1 | 23 June 2003 |
| | | | | AU | 2002359489 | B2 | 30 October 2008 |
| | | | | NO | 20042634 | L | 23 June 2004 |
| CN | 102838539 | A | 26 December 2012 | None | | | |
| CN | 102724970 | A | 10 October 2012 | WO | 2011055303 | A1 | 12 May 2011 |
| | | | | ES | 2757882 | T3 | 30 April 2020 |
| | | | | ES | 2757882 | T5 | 10 May 2023 |
| | | | | HUE | 046606 | T2 | 30 March 2020 |
| | | | | EP | 3566697 | A1 | 13 November 2019 |
| | | | | EP | 4066821 | A1 | 05 October 2022 |
| | | | | CY | 1122330 | T1 | 27 January 2021 |
| | | | | US | 2014004184 | A1 | 02 January 2014 |
| | | | | US | 8790708 | B2 | 29 July 2014 |
| | | | | US | 2011111018 | A1 | 12 May 2011 |
| | | | | US | 8518446 | B2 | 27 August 2013 |
| | | | | PL | 2498756 | T3 | 31 March 2020 |
| | | | | PL | 2498756 | T5 | 17 April 2023 |
| | | | | DK | 2498756 | T3 | 25 November 2019 |
| | | | | DK | 2498756 | T4 | 20 March 2023 |
| | | | | PT | 2498756 | T | 26 November 2019 |
| | | | | CA | 2780428 | A1 | 12 May 2011 |
| | | | | CA | 2780428 | C | 13 February 2018 |
| | | | | AU | 2010316683 | A1 | 24 May 2012 |
| | | | | AU | 2010316683 | B2 | 08 October 2015 |

Form PCT/ISA/210 (patent family annex) (July 2022)

**INTERNATIONAL SEARCH REPORT**
**Information on patent family members**

International application No.
**PCT/CN2023/080921**

| Patent document cited in search report | Publication date (day/month/year) | Patent family member(s) | Publication date (day/month/year) |
|---|---|---|---|
| | | JP 2011098964 A | 19 May 2011 |
| | | JP 5835883 B2 | 24 December 2015 |
| | | AR 078952 A1 | 14 December 2011 |
| | | SI 2498756 T1 | 31 January 2020 |
| | | SI 2498756 T2 | 28 April 2023 |
| | | JP 2015091882 A | 14 May 2015 |
| | | TW 201138774 A | 16 November 2011 |
| | | TWI 466690 B | 01 January 2015 |
| | | JP 2017075192 A | 20 April 2017 |
| | | HRP 20192026 T1 | 07 February 2020 |
| | | HRP 20192026 T4 | 31 March 2023 |
| | | LT 2498756 T | 10 December 2019 |
| | | FI 2498756 T4 | 22 March 2023 |
| | | AR 114143 A2 | 29 July 2020 |
| | | NZ 599763 A | 27 June 2014 |
| | | RS 59599 B1 | 31 January 2020 |
| | | RS 59599 B2 | 31 March 2023 |
| | | EP 2498756 A1 | 19 September 2012 |
| | | EP 2498756 B1 | 04 September 2019 |
| | | EP 2498756 B2 | 15 February 2023 |
| CN 103242273 A | 14 August 2013 | BR 112014019402 A2 | 20 June 2017 |
| | | BR 112014019402 A8 | 11 July 2017 |
| | | BR 112014019402 B1 | 15 December 2020 |
| | | RU 2014132159 A | 27 March 2016 |
| | | RU 2583900 C2 | 10 May 2016 |
| | | US 2015018542 A1 | 15 January 2015 |
| | | US 9533965 B2 | 03 January 2017 |
| | | EP 2813495 A1 | 17 December 2014 |
| | | EP 2813495 A4 | 17 June 2015 |
| | | EP 2813495 B1 | 28 September 2016 |
| | | CA 2863988 A1 | 15 August 2013 |
| | | CA 2863988 C | 22 March 2016 |
| | | JP 2015511942 A | 23 April 2015 |
| | | JP 5926821 B2 | 25 May 2016 |
| | | WO 2013117120 A1 | 15 August 2013 |
| CN 102209714 A | 05 October 2011 | JP 2015110633 A | 18 June 2015 |
| | | JP 6047184 B2 | 21 December 2016 |
| | | KR 20110089418 A | 08 August 2011 |
| | | KR 101749192 B1 | 20 June 2017 |
| | | AU 2009312464 A1 | 14 May 2010 |
| | | AU 2009312464 A8 | 06 September 2012 |
| | | AU 2009312464 B2 | 16 April 2015 |
| | | NZ 592617 A | 28 February 2014 |
| | | ZA 201104298 B | 28 March 2012 |
| | | IL 212726 A0 | 31 July 2011 |
| | | IL 212726 A | 30 March 2017 |
| | | CA 2741990 A1 | 14 May 2010 |
| | | CA 2741990 C | 07 February 2017 |
| | | SMT 201700006 B | 08 March 2017 |
| | | EP 2364302 A2 | 14 September 2011 |

Form PCT/ISA/210 (patent family annex) (July 2022)

**INTERNATIONAL SEARCH REPORT**
**Information on patent family members**

International application No.
**PCT/CN2023/080921**

| Patent document cited in search report | Publication date (day/month/year) | Patent family member(s) | | | Publication date (day/month/year) |
|---|---|---|---|---|---|
| | | EP | 2364302 | B1 | 05 October 2016 |
| | | SI | 2364302 | T1 | 31 January 2017 |
| | | RU | 2011114559 | A | 20 December 2012 |
| | | RU | 2537945 | C2 | 10 January 2015 |
| | | WO | 2010052569 | A2 | 14 May 2010 |
| | | WO | 2010052569 | A3 | 16 December 2010 |
| | | WO | 2010052569 | A8 | 03 November 2011 |
| | | CY | 1118438 | T1 | 28 June 2017 |
| | | JP | 2012508223 | A | 05 April 2012 |
| | | JP | 5738768 | B2 | 24 June 2015 |
| | | LT | 2364302 | T | 25 January 2017 |
| | | HRP | 20161769 | T1 | 24 February 2017 |
| | | GB | 0821219 | D0 | 31 December 2008 |
| | | GB | 2465405 | A | 19 May 2010 |
| | | ES | 2609296 | T3 | 19 April 2017 |
| | | BRPI | 0921709 | A2 | 25 August 2015 |
| | | BRPI | 0921709 | B1 | 04 October 2022 |
| | | MX | 2011004889 | A | 29 February 2012 |
| | | DK | 2364302 | T3 | 23 January 2017 |
| | | US | 2011275762 | A1 | 10 November 2011 |
| | | US | 8921361 | B2 | 30 December 2014 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 102724970
- CN 106913529
- US 6002008 A
- US 6288082 B
- US 6297258 B
- US 6384051 B
- US 7399865 B
- CN 103242273